# FASCICULE DE BREVET EUROPEEN

(11) **EP 4 466 117 B1**
(45) Date de publication et mention de la délivrance du brevet: **08.04.2026**
(21) Numéro de dépôt: 23704828.5
(22) Date de dépôt: 20.01.2023
(51) Int. Cl.: B25J 9/00

(54) **EXOSQUELETTE D'ASSISTANCE AU MAINTIEN EN POSTURE PENCHÉE EN AVANT ET AU REDRESSEMENT**
EXOSKELETT ZUR UNTERSTÜTZUNG DER AUFRECHTERHALTUNG EINER NACH VORNE GENEIGTEN HALTUNG UND BEIM BEGRADIGEN
EXOSKELETON FOR ASSISTANCE IN MAINTAINING A LEANING FORWARD POSTURE AND IN STRAIGHTENING UP

(30) Priorité: 21.01.2022 FR 2200512
(43) Date de publication de la demande: 27.11.2024
(73) Titulaire: Robotiques 3 Dimensions, 89470 Moneteau (FR)
(72) Inventeur: GRYGOROWICZ, Serge, 89580 GY L'EVEQUE (FR)
(74) Mandataire: Cabinet Chaillot
(86) Numéro de dépôt international: PCT/IB2023/050508
(87) Numéro de publication internationale: WO 2023/139537

(56) Documents cités:
- WO-A1-2021/234061
- CN-B- 106 514 628
- DE-B4- 102004 008 509
- US-A1- 2012 184 881
- US-A1- 2015 306 762
- US-A1- 2018 280 178
- US-A1- 2021 298 985
- US-B2- 10 376 402

## Description

La présente invention se rapporte au domaine des dispositifs d'assistance physique de type exosquelette destinés à être portés par un utilisateur.

La présente invention porte plus particulièrement sur un exosquelette d'assistance au maintien de l'utilisateur en posture penchée en avant et d'aide au redressement de l'utilisateur vers la position debout, l'assistance étant assurée par la génération d'un couple de redressement par l'exosquelette.

Notamment, mais non limitativement, la présente invention intéresse le domaine de la viticulture.

En effet, le travail de la vigne implique de rester, éventuellement de manière prolongée, dans une posture penchée en avant, avec les jambes pliées ou non, mettant le dos du travailleur à rude épreuve, tout particulièrement dans sa partie lombaire. Il en résulte souvent des troubles musculo-squelettiques, tels que fatigues et douleurs dorsales, pouvant se transformer, à la longue, en lombalgie sévère, se traduisant en maladie professionnelle, exigeant parfois le recours à la chirurgie pour leur traitement.

De telles situations de travail en position penchée en avant se rencontrent également fréquemment dans les domaines du maraîchage et de l'horticulture.

De nombreux dispositifs d'assistance physique de type exosquelette ont été créés depuis plusieurs décennies pour améliorer ces conditions de travail et soulager le dos d'un opérateur qui travaille en étant penché vers l'avant.

Selon une première approche, une première distinction peut être effectuée parmi ces exosquelettes, entre les exosquelettes dits passifs et les exosquelettes dits actifs.

Les exosquelettes passifs génèrent une assistance uniquement basée sur des éléments mécaniques, comme des ressorts, des élastiques ou des lames élastiques déformables. Leur fonctionnement repose sur le stockage et la dissipation de l'énergie mécanique fournie par l'utilisateur lorsqu'il se penche, et donc ne nécessite aucune source d'énergie extérieure telle que des batteries. On peut citer, par exemple, les brevets américains US1,409,326 et US4,829,989 qui utilisent une résistance passive à ressort pour créer un couple résistant, ou autrement dit un couple de redressement, entre le buste et les jambes de l'utilisateur. Il en résulte une simplicité de fabrication et d'entretien, ainsi qu'une facilité d'usage. Les exosquelettes passifs présentent également les avantages d'être légers et de coût attractif puisqu'ils n'intègrent pas de composants onéreux. Cependant, leur adaptabilité est réduite, ce qui est un inconvénient lorsque les conditions de travail sont variables.

Les exosquelettes actifs sont eux équipés d'actionneurs générant un effort à partir d'une source d'énergie extérieure. Leur conception est généralement plus complexe et coûteuse, et leur poids est élevé, notamment en raison de la masse des actionneurs, du stockage d'énergie, de la centrale de contrôle et des capteurs. Cependant, ils offrent une meilleure versatilité. Les efforts générés peuvent être commandés et ajustés en temps réel, pouvant ainsi s'adapter aux variations de fréquence, d'amplitude, de vitesse, etc.

Selon une autre approche, une seconde distinction peut être effectuée entre les exosquelettes rigides et les exosquelettes souples.

Les exosquelettes rigides utilisent des matériaux de faible densité, tels que les matières plastiques, les composites en fibre de carbone ou certains métaux tels que l'aluminium et le titane. Leur rigidité permet une transmission d'efforts importante, généralement orthogonalement au corps de l'utilisateur, au détriment d'un poids généralement plus élevé. On peut citer la demande internationale de brevet WO2021187973 qui décrit un exosquelette rigide qui supporte le buste par transmission des efforts aux cuisses. Pour cela, des palettes sont positionnées sur les cuisses et des systèmes de cames compriment des ressorts. Le couple de redressement ainsi généré est transmis au buste par un corps en appui direct contre le buste.

Les exosquelettes souples ont une structure assemblée à partir de matériaux très flexibles, notamment textiles, qui se déforment en cours d'utilisation. Ces exosquelettes souples sont légers et épousent le corps de l'utilisateur. Leur faible masse et leur position proche du corps font que l'inertie de la structure a un impact minimal sur la cinématique naturelle de l'utilisateur. Cependant, pour une déformation d'amplitude donnée, les efforts transmissibles, généralement tangentiels au corps de l'utilisateur, restent faibles.

La Société déposante a cherché à proposer une solution d'assistance physique de type exosquelette combinant les avantages de chacun de ces types d'exosquelette, à savoir ceux des exosquelettes actifs, passifs, rigides et souples.

En particulier, la présente invention vise à proposer un exosquelette léger, adaptable à la morphologie des utilisateurs, de conception simple, commode à utiliser (pas d'éléments techniques sur les côtés de l'utilisateur), d'un coût raisonnable, au besoin capable de fonctionner sans source d'énergie extérieure, sans capteur et sans motorisation, autrement dit dans un mode de fonctionnement passif, et apte à générer des efforts importants orthogonalement au corps de l'utilisateur.

Dans les exosquelettes passifs de l'état antérieur connu, tel que le brevet américain US1,409,326, le couple de redressement créé entre le buste et les jambes de l'utilisateur, lorsque l'utilisateur portant l'exosquelette se penche en avant, est directement proportionnel à l'effort de déformation en flexion de ressorts à lames, lequel effort est la résistance que l'exosquelette oppose à l'utilisateur qui se penche. On peut également citer l'exosquelette conçu par l'université Virginia Tech et l'entreprise Lowe's ( https://vtx.vt.edu/articles/2017/05/eng-lowesexosuit.html), dans lequel des éléments flexibles en fibre de carbone sont déformés en flexion et se tendent comme un arc lorsque l'utilisateur se penche en avant. Ainsi, le couple de redressement généré par ces exosquelettes suit une loi linéaire et augmente proportionnellement à l'augmentation de l'angle d'inclinaison entre le buste et les jambes. Il en résulte que l'effort que l'utilisateur devra fournir pour se pencher en avant sera de plus en plus important à mesure que l'angle entre son dos et l'arrière de ses cuisses augmentera.

WO 2021/234061 A1 et CN 106 514 628 B divulguent des exosquelettes selon le préambule de la revendication 1. D'autres exosquelettes connus sont également décrits dans US 2018/280178 A1, US 2021/298985 A1, US 2012/184881 A1, US 10 376 402 B2, US 2015/306762 A1 et DE 10 2004 008509 B4.

La Société déposante a également cherché à proposer une solution permettant de pallier cet inconvénient, et donc permettant à un utilisateur qui porte l'exosquelette selon la présente invention de se pencher en avant jusqu'à un certain angle prédéterminé en étant soutenu par l'exosquelette, et de se pencher au-delà de cet angle prédéterminé, par exemple afin d'aller jusqu'au sol, sans subir une augmentation de la résistance que lui oppose l'exosquelette, mais au contraire une diminution de cette résistance.

La solution selon la présente invention repose sur le fait que le couple de redressement généré par l'exosquelette dans un mode de fonctionnement passif dépend d'un effort de déformation rectiligne, et non plus d'un effort de déformation en flexion d'un élément mécanique, et n'est pas directement proportionnel à cet effort de déformation, de sorte que le couple résistant généré ne suit plus une loi linéaire, mais une loi sinusoïdale ou pseudo-sinusoïdale.

La présente invention a ainsi pour objet un exosquelette d'assistance au maintien en posture penchée en avant et au redressement d'un utilisateur, l'exosquelette comprenant une structure dorsale configurée pour être fixée de façon amovible au niveau de la région dorsale de l'utilisateur et deux structures de liaison de cuisse configurées pour être fixées de façon amovible aux cuisses de l'utilisateur et reliées à la structure dorsale par l'intermédiaire d'un dispositif de génération de couple passif comprenant, pour chaque structure de liaison de cuisse, un support supérieur couplé à la structure dorsale, un support inférieur couplé à la structure de liaison de cuisse, le support supérieur et le support inférieur étant articulés l'un à l'autre en rotation, dans un même plan, autour d'un axe d'articulation, caractérisé par le fait que le dispositif de génération de couple comprend en outre, pour chaque structure de liaison de cuisse, un élément de transmission longitudinal, souple et inextensible, ayant une première extrémité reliée, en un premier point de fixation, au support inférieur, et une seconde extrémité reliée, en un second point de fixation, à une première partie, dite mobile, d'un organe élastiquement déformable dont une seconde partie, dite fixe, est solidaire en rotation du support supérieur de façon à tourner conjointement avec le support supérieur relativement au support inférieur, l'organe élastiquement déformable étant apte à produire une force rectiligne le long d'un axe de déformation perpendiculaire à l'axe d'articulation et dont la grandeur est linéairement proportionnelle à sa déformation élastique, l'élément de transmission étant par ailleurs en appui contre un premier point d'appui solidaire du support supérieur et un second point d'appui solidaire du support inférieur, chacun des premier et second points d'appui se trouvant à une distance fixe de l'axe d'articulation lors d'une rotation entre le support supérieur et le support inférieur, le dispositif de génération de couple étant ainsi apte, dans un mode de fonctionnement passif, lorsque l'utilisateur se penche en avant, dans un plan perpendiculaire à l'axe d'articulation, à produire un couple de redressement appliqué entre la structure dorsale et chaque structure de liaison de cuisse du fait de la déformation de l'organe élastiquement déformable par l'élément de transmission, lequel couple de redressement suit une loi pseudo-sinusoïdale qui est fonction de la force produite par l'organe élastiquement déformable et du sinus d'un angle formé entre la droite reliant l'axe d'articulation et le premier point d'appui et la droite reliant les premier et second points d'appui.

Un tel exosquelette peut au besoin fonctionner sans autre source d'énergie que celle de l'utilisateur qui le porte. Dans un tel cas, en raison du fait qu'en utilisation la longueur de l'élément de transmission entre les premier et second points de fixation est fixe, c'est l'inclinaison entre le tronc et les cuisses de l'utilisateur qui provoque la rotation entre chaque support supérieur et le support inférieur associé autour de l'axe d'articulation et donc le déplacement de l'élément de transmission associé qui à son tour provoque la déformation de l'organe élastiquement déformable, la force produite par cette déformation générant le couple de redressement. Le fait que ce couple de redressement suit une loi pseudo-sinusoïdale, et non une loi linéaire, permet à un utilisateur de se pencher jusqu'à un certain angle d'inclinaison, par exemple 90 degrés, en étant soutenu par l'exosquelette, et de se pencher au-delà de cet angle d'inclinaison, par exemple au-delà de 90 degrés, sans subir une augmentation de la résistance, et même au contraire une diminution de la résistance. Par ailleurs, la structure dorsale et chaque structure de liaison de cuisse étant montées à rotation, par l'intermédiaire des supports supérieur et inférieur, autour de l'axe articulation sur lequel est appliqué le couple de redressement, la structure dorsale et les structures de liaison de cuisse sont amenées, en utilisation, à appliquer une force de résistance respectivement sur le tronc et les cuisses de l'utilisateur, lesquelles forces de résistance sont orthogonales au tronc et aux cuisses, respectivement et donc plus facilement tolérés que s'il s'agissait de forces tangentielles.

On souligne ici que les première et seconde extrémités de l'élément de transmission peuvent être reliées de manière directe ou de manière indirecte, autrement dit par l'intermédiaire d'une ou plusieurs pièces distinctes, au support inférieur et à l'organe élastiquement déformable, respectivement.

De préférence, chaque élément de transmission est un câble, notamment un câble réalisé en fibre de polyéthylène haut module, comme par exemple commercialisé sous la dénomination Dyneema^{®}.

Un tel matériau est léger, très résistant, en particulier à la torsion et à l'abrasion, et a une très faible élongation.

Avantageusement, la longueur de chaque élément de transmission est telle qu'en utilisation, jusqu'à un angle d'inclinaison prédéterminé du dos de l'utilisateur par rapport à l'arrière de ses cuisses, l'élément de transmission ne provoque pas de déformation de l'organe élastiquement déformable. En particulier, cette longueur peut être choisie de manière à permettre de ne pas déformer l'organe élastiquement déformable lors de la marche de l'utilisateur.

Autrement dit, la longueur libre de chaque élément de transmission pourra être avantageusement supérieure à la somme de la distance entre le premier point de fixation et le second point d'appui, de la distance entre les premier et second points d'appui et de la distance entre le premier point d'appui et le second point de fixation, au moins lorsque l'angle de pivotement relatif des supports supérieur et inférieur est nul, négatif ou faible, un tel angle étant typiquement rencontré lors de la marche, et donc pour un angle qui ne correspond pas au passage de l'utilisateur en posture penchée.

Ladite longueur libre de l'élément de transmission peut être définie à l'avance à une valeur fixe.

De préférence, cette longueur libre d'élément de transmission sera rendue disponible à l'utilisateur lorsque ce dernier le souhaite. Ainsi, avantageusement, l'exosquelette comprend, pour chaque élément de transmission, un module de mise en traction/ détente auquel est relié l'élément de transmission, le module de mise en traction/ détente étant actionnable par l'utilisateur de façon à faire varier la longueur libre de l'élément de transmission, le module de mise en traction/détente étant mobile entre une position embrayée, dans laquelle l'élément de transmission est tendu, et une position débrayée, dans laquelle l'élément de transmission est détendu, de telle sorte qu'il ne provoque pas de déformation de l'organe élastiquement déformable. Ainsi, la position débrayée permet à un utilisateur de s'équiper de l'exosquelette de manière aisée, un mouvement libre vers l'avant des structures de liaison de cuisse étant autorisé.

On entend par « tendu » que l'élément de transmission est mis en traction et « détendu » qu'il n'est pas soumis à une force de traction.

La variation de la longueur libre peut par exemple être obtenue par déplacement du premier point de fixation. Selon un mode de réalisation particulier, le module de mise en traction/détente est formé par un tambour d'enroulement sur lequel le premier point de fixation est positionné, le tambour d'enroulement étant relié au support inférieur de façon à être apte à tourner autour d'un axe parallèle audit axe d'articulation et ainsi à enrouler ou dérouler l'élément de transmission, selon le sens de rotation, le module de mise en traction/détente comprenant en outre des moyens de blocage du module de mise en traction/détente dans chacune des positions embrayée et débrayée.

Bien entendu, l'on pourrait prévoir d'autres formes de réalisation pour le module de mise en traction/détente, comme par exemple une vis sans fin dont une extrémité porte le premier point de fixation et qui est déplaçable en translation.

Le module de mise en traction/détente pourra être déplaçable manuellement, et ainsi comprendre un organe d'actionnement accessible à l'utilisateur, ou bien être déplaçable par des moyens motorisés, par exemple pour faire tourner le tambour d'enroulement ou déplacer en translation la vis sans fin. De tels moyens motorisés pourront être commandés par l'utilisateur, par exemple par l'appui sur un bouton porté par l'exosquelette, ou bien leur commande pourra être automatisée de façon à tenir compte de l'angle d'inclinaison de l'utilisateur, mesuré par des moyens connus en soi, tels qu'une centrale inertielle.

Dans un mode de réalisation préféré, chaque support inférieur comporte une fourchette de réglage de couple sur laquelle est positionné le second point d'appui, et dont la position est réglable par rapport à l'axe d'articulation, de telle sorte que le réglage de la position de la fourchette permet de modifier la distance entre l'axe d'articulation et le second point d'appui.

Ce réglage permet de modifier le couple de redressement appliqué par l'exosquelette et permet donc à l'exosquelette de s'adapter bio-mécaniquement au besoin de soutien qui varie suivant la taille et la corpulence de l'utilisateur.

Avantageusement, le dispositif de génération de couple comprend des éléments de guidage de la déformation du ou des organes élastiquement déformables, comme par exemple des éléments de guidage en translation de la partie mobile de l'organe élastiquement déformable à laquelle l'élément de transmission est relié.

Dans un mode de réalisation particulier de l'invention, le dispositif de génération de couple comprend deux organes élastiquement déformables, à savoir un organe élastiquement déformable pour chaque structure de liaison de cuisse.

De préférence, chaque organe élastiquement déformable comprend un ressort de compression, chaque ressort de compression ayant une extrémité inférieure qui constitue ladite partie fixe et est fixée au support supérieur, et une extrémité supérieure qui constitue ladite partie mobile et est reliée à l'élément de transmission, au niveau du second point de fixation, chaque ressort s'étendant selon un axe de déformation parallèle à l'axe de la structure dorsale et perpendiculaire à l'axe d'articulation, de telle sorte qu'en utilisation, lorsque l'utilisateur se penche en avant, le second point de fixation de l'élément de transmission se déplace en direction de l'extrémité inférieure du ressort d'une distance, provoquant la compression du ressort, qui amène le ressort à exercer une force de rappel axiale proportionnelle à la distance de déplacement.

L'utilisation de ressorts de compression est particulièrement économique. En variante, l'organe élastiquement déformable pourrait être un câble élastique, du type sandow, un ressort de traction, ou encore un ressort à gaz.

De préférence, un fourreau s'étend autour du ou de chaque organe élastiquement déformable. Cela permet d'améliorer la sécurité de fonctionnement.

Le ou chaque organe élastiquement déformable peut, par exemple, avoir une constante de rappel de 17,6 N/mm.

Selon un mode de réalisation particulier, l'exosquelette comprend en outre un module électronique de pilotage du couple de redressement, comprenant des moyens de détermination de l'angle d'inclinaison du buste de l'utilisateur, un actionneur motorisé configuré pour commander, lors de son actionnement, dans un mode de fonctionnement dit quasi-passif de l'exosquelette, la déformation de l'organe élastiquement déformable, indépendamment d'un quelconque pivotement relatif entre les supports supérieur et inférieur, des moyens de commande de l'actionneur sur la base de l'angle d'inclinaison déterminé par lesdits moyens de détermination, et des moyens de maintien de l'organe élastiquement déformable dans sa configuration déformée par l'actionneur.

L'exosquelette selon la présente invention peut alors être utilisé dans un mode de fonctionnement dit quasi-passif, dans lequel l'énergie requise pour la déformation de l'organe élastiquement déformable est fournie par l'actionneur, et non plus par l'utilisateur comme dans le mode de fonctionnement passif. Cependant, contrairement aux exosquelettes actifs, l'énergie fournie par l'actionneur est stockée dans l'organe élastiquement déformable et elle n'est donc pas fournie de manière continue. L'exosquelette selon la présente invention ne présente donc pas les inconvénients associés à la consommation de l'énergie électrique que l'on rencontre dans les exosquelettes actifs.

De préférence, lesdits moyens de maintien sont simplement l'élément de transmission relié à l'organe élastiquement déformable respectif.

Le module de pilotage peut alors être configuré pour simplement assurer la même déformation de l'organe élastiquement déformable que celle obtenue par l'utilisateur se penchant en avant, jusqu'à par exemple un angle prédéterminé, tel qu'un angle de 45°, puis fonctionner de la même manière que dans le mode de fonctionnement passif, avec les mêmes avantages.

Selon un mode de réalisation particulier, le module de pilotage est configuré pour produire une déformation de l'organe élastiquement déformable qui est supérieure à celle produite dans le mode de fonctionnement passif, pour un même angle d'inclinaison du buste de l'utilisateur, puis, à partir d'un angle d'inclinaison prédéterminé, relâcher, éventuellement de manière progressive, à l'aide desdits moyens de maintien, l'organe élastiquement déformable. De cette manière, l'exosquelette peut appliquer à l'utilisateur un couple de redressement supérieur à celui que sa morphologie autorise et à des angles d'inclinaison plus faibles. Ceci convient particulièrement par exemple au travail dans une posture penchée à 45° ou moins, où le couple de redressement ne pourrait pas soulager suffisamment l'utilisateur dans le mode de fonctionnement passif où il suit une loi pseudo-sinusoïdale.

L'actionneur motorisé peut par exemple comporter le tambour d'enroulement ou la vis discutés ci-dessus et constituant le module de mise en traction/détente, et ainsi la déformation de l'organe élastiquement déformable est obtenue par traction de l'élément de transmission.

Bien entendu, l'on pourra prévoir tout autre actionneur motorisé, notamment en fonction de l'organe élastiquement déformable qui sera utilisé. L'on pourrait donc prévoir que la déformation de l'organe élastiquement déformable ne soit pas obtenue par traction de l'élément de transmission, mais par exemple par poussée ou traction directement sur l'organe élastiquement déformable. Par exemple, si l'organe élastiquement déformable est un ressort de compression, l'actionneur motorisé peut comprendre une vis sans fin, qui est entraînée en rotation par un moteur électrique et dont l'axe longitudinal est coaxial à l'axe de déformation du ressort de compression, et un écrou qui est monté sur la vis sans fin de façon à être déplaçable en translation par rotation de celle-ci et qui est en contact, éventuellement par l'intermédiaire d'une jupe allongée, contre l'extrémité du ressort de compression.

Lesdits moyens de détermination sont par exemple constitués par une centrale inertielle. Lesdits moyens de commande peuvent par exemple être constitués par tout moyen électronique approprié, comme un circuit électronique de traitement, tel qu'un processeur, un microprocesseur, un microcontrôleur, un processeur de signaux numériques (DSP), ou un composant logique programmable de type matrice prédiffusée programmable (FPGA) ou composant à application spécifique (ASIC), associé à de la mémoire.

De préférence, la structure dorsale comprend deux paires de tubes télescopiques dorsaux, chaque paire comprenant un tube inférieur et un tube supérieur apte à coulisser dans le tube inférieur, le tube inférieur de chaque paire étant solidaire du support supérieur correspondant, le tube supérieur de chaque paire étant couplé à des moyens de fixation amovible à l'épaule de l'utilisateur, les deux paires s'étendant selon des axes parallèles entre eux et parallèles à l'axe de déformation.

Une telle structure télescopique permet d'adapter l'exosquelette à la taille du dos de l'utilisateur.

De préférence, pour chaque paire de tubes télescopiques dorsaux, le tube inférieur est monté par une liaison pivot glissant sur le tube supérieur. Une telle liaison permet à un utilisateur d'effectuer librement un mouvement de pivotement de ses épaules par rapport au bas de son dos.

De préférence, chaque structure liaison de cuisse comprend une paire de tubes télescopiques de cuisse, à savoir un tube supérieur et un tube inférieur apte à coulisser dans le tube supérieur, le tube supérieur étant solidaire du support inférieur correspondant et le tube inférieur étant couplé à des moyens de fixation amovible à la cuisse de l'utilisateur.

Une telle structure télescopique permet d'adapter l'exosquelette à la longueur des cuisses de l'utilisateur.

De préférence, pour chaque paire de tubes télescopiques de cuisse, le tube supérieur est monté par une liaison pivot glissant sur le tube inférieur. Une telle liaison permet à un utilisateur d'effectuer librement un mouvement de pivotement de ses cuisses par rapport au bas de son dos.

De préférence, chaque structure de liaison de cuisse est couplée au support inférieur correspondant de telle sorte que la paire de tubes télescopiques de cuisse s'étend dans le prolongement de la paire de tubes télescopiques dorsaux correspondants. Ainsi, en utilisation, chaque paire de tubes télescopiques de cuisse est positionné en regard de l'arrière de la cuisse et ne dépasse pas sur les côtés de l'utilisateur. Par conséquent, l'utilisateur qui porte l'exosquelette n'est pas gêné dans ses mouvements et l'exosquelette ne peut pas entrer en collision avec l'environnement situé de part et d'autre de l'utilisateur.

De préférence, l'exosquelette comprend en outre un harnais textile comprenant au moins deux bretelles reliées à la structure dorsale pour la fixation amovible de la structure dorsale aux épaules de l'utilisateur, une ceinture reliée au niveau des supports supérieurs pour la fixation amovible des support supérieurs au niveau de la région lombaire de l'utilisateur, et deux sangles de cuisse reliées à chaque structure de liaison de cuisse pour la fixation amovible des structures de liaison de cuisse aux cuisses de l'utilisateur.

Le harnais textile permet d'ajuster l'exosquelette sur le corps de l'utilisateur par de simples réglages de longueurs de boucles et de sangles.

De préférence, l'exosquelette comprend en outre, pour chaque structure de liaison de cuisse, un appui-fesses couplé au support inférieur par l'intermédiaire d'une liaison rotule.

Un coussin de fesses peut être couplé à l'appui-fesses, de sorte que lorsque le harnais est porté par un utilisateur, le coussin de fesses est interposé entre les fesses de l'utilisateur et chaque appui-fesses.

De plus, les sangles de cuisse peuvent également être munies chacune d'un coussin de cuisse.

Pour chaque structure de liaison de cuisse, chacun du support supérieur et du support inférieur pourra être formé par un ensemble rigide, c'est-à-dire non articulé, en ce sens qu'en utilisation, lorsque l'utilisateur se penche en avant, les pièces qui composeraient le support supérieur ou le support inférieur ne pivotent pas les unes par rapport aux autres de façon à influer sur le couple de redressement.

Selon un mode de réalisation particulier, avantageux, chaque support inférieur est un support articulé, comprenant un corps de base, couplé à la structure de liaison de cuisse et auquel se situe le premier point de fixation de l'élément de transmission, et un ensemble d'articulation à maillon(s) dont une première extrémité est articulée en rotation au support supérieur autour de l'axe d'articulation et dont une seconde extrémité est articulée en rotation au corps de base autour d'un axe d'articulation secondaire parallèle audit axe d'articulation, le second point d'appui étant solidaire de l'ensemble d'articulation à maillon(s), dans la région de sa première extrémité, l'élément de transmission étant par ailleurs en appui contre un troisième point d'appui solidaire de l'ensemble d'articulation à maillon(s), dans la région de sa seconde extrémité, et contre un quatrième point d'appui solidaire du corps de base, chacun des troisième et quatrième points d'appui se trouvant à une distance fixe de l'axe d'articulation secondaire lors d'une rotation entre le support supérieur et le support inférieur.

L'ensemble d'articulation à maillon(s) agit à la manière d'une ou plusieurs vertèbres et permet de réduire l'encombrement du dispositif de génération de couple et donc la place occupée par l'exosquelette au niveau des fesses de l'utilisateur, pour par exemple lui permettre de s'asseoir plus facilement sur un siège, comme notamment pour conduire un chariot élévateur, et ceci tout en conservant un couple de redressement qui suit une loi pseudo-sinusoïdale dépendant, entre autres, de la force produite par l'organe élastiquement déformable et du sinus de l'angle formé entre la droite reliant l'axe d'articulation et le premier point d'appui et la droite reliant les premier et second points d'appui, comme dans le cas de supports supérieur et inférieur rigides.

Chaque ensemble d'articulation à maillon(s) peut comprendre un seul maillon rigide dont une première extrémité est articulée en rotation au support supérieur autour de l'axe d'articulation et dont une seconde extrémité est articulée en rotation au corps de base autour d'un axe d'articulation secondaire parallèle audit axe d'articulation, le maillon portant les second et troisième points d'appui.

En variante, chaque ensemble d'articulation à maillon(s) comprend au moins deux maillons rigides successifs articulés entre eux, un premier des maillons, dit maillon supérieur, ayant une première extrémité articulée en rotation au support supérieur autour de l'axe d'articulation, et un second des maillons, dit maillon inférieur, ayant une seconde extrémité articulée en rotation au corps de base autour d'un axe d'articulation secondaire parallèle audit axe d'articulation, chaque maillon étant par ailleurs articulé en rotation au maillon suivant autour d'un axe d'articulation intermédiaire parallèle audit axe d'articulation, le maillon supérieur portant le second point d'appui et le maillon inférieur portant le troisième point d'appui, l'élément de transmission étant par ailleurs en appui, pour chaque paire de maillons successifs, composée d'un premier maillon et d'un second maillon, contre un premier point d'appui intermédiaire solidaire du premier maillon de ladite paire, dans la région de sa seconde extrémité, et contre un second point d'appui intermédiaire solidaire du second maillon de ladite paire, dans la région de sa première extrémité, chacun des premier et second points d'appui intermédiaires se trouvant à une distance fixe de l'axe d'articulation intermédiaire entre les premier et second maillons de ladite paire lors d'une rotation entre le support supérieur et le support inférieur.

Les différents points d'appui, qu'ils soient intermédiaires ou non, peuvent être formés par des poulies contre lesquelles l'élément de transmission est en contact. Le quatrième point peut toutefois être le premier point de fixation de l'élément de transmission.

Pour mieux illustrer l'objet de la présente invention, on va en décrire ci-après, à titre illustratif et non limitatif, un mode de réalisation particulier, avec référence aux dessins annexés.

Sur ces dessins :
[Fig.1] est une vue d'ensemble de l'exosquelette selon un premier mode de réalisation particulier de la présente invention, porté par utilisateur ;
[Fig.2] est une vue représentant les forces appliquées par l'exosquelette de la [Fig.1] sur l'utilisateur lorsque celui-ci se penche en avant, l'exosquelette n'étant pas représenté pour plus de clarté ;
[Fig.3] est une vue en perspective de l'exosquelette de la [Fig.1], le harnais n'étant pas représenté ;
[Fig.4] est une vue de côté, en coupe longitudinale, du dispositif passif de génération de couple de l'exosquelette de la [Fig.3], dans une première position ;
[Fig.5] est une vue de côté, en coupe longitudinale, du dispositif passif de génération de couple de l'exosquelette de la [Fig.3], dans une deuxième position ;
[Fig.6] est une vue en perspective d'un support supérieur du dispositif de génération de couple de la [Fig.3] ;
[Fig.7] est une vue en perspective d'un support inférieur du dispositif de génération de couple de la [Fig.3] ;
[Fig.8] est une vue en perspective d'une fourchette de réglage de couple du dispositif de génération de couple de la [Fig.3] ;
[Fig.9] est un schéma représentant les paramètres entrant dans le calcul du couple de redressement appliqué autour l'axe d'articulation ;
[Fig.10] est une courbe montrant la valeur du couple de redressement généré par l'exosquelette en fonction de l'angle α de la [Fig.9], la fourchette de réglage de couple étant dans une première position ; et
[Fig.11] est une autre courbe montrant la valeur du couple de redressement généré par l'exosquelette en fonction de l'angle α, la fourchette de réglage de couple étant dans une deuxième position ;
[Fig.12] est une vue schématique d'un module de pilotage du couple de redressement d'un exosquelette selon une variante du premier mode de réalisation particulier de la présente invention ;
[Fig.13] est une vue en perspective d'un exosquelette selon un second mode de réalisation de la présente invention, le harnais n'étant pas représenté ;
[Fig.14] est une vue en perspective du dispositif de génération de couple de l'exosquelette de la [Fig.13] ;
[Fig.15] est une vue en coupe longitudinale du dispositif de génération de couple de la [Fig.13], en position au repos ;
[Fig.16] est une vue analogue à la [Fig.15], en position de travail ; et
[Fig.17] est une vue analogue à la [Fig.16] pour une variante du second mode de réalisation.

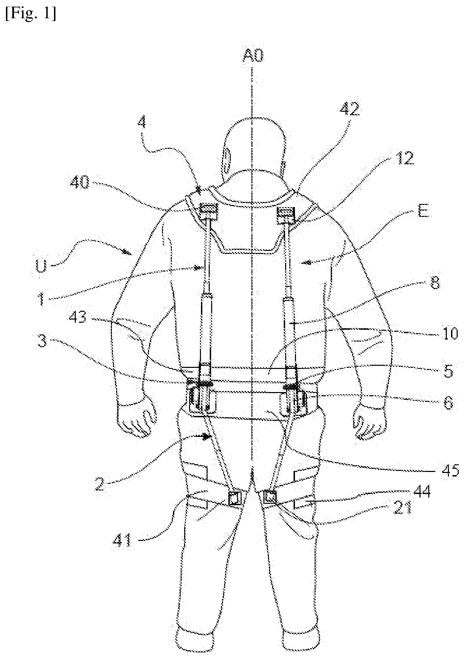

Si l'on se réfère tout d'abord à la [Fig.1], on peut voir que l'exosquelette E selon un premier mode de réalisation de la présente invention est destiné à être porté par un utilisateur U, tel qu'un viticulteur, un maraîcher, un horticulteur, un jardinier, ou toute personne travaillant en position courbée de manière prolongée. Par utilisateur U on entend toute personne qui porte l'exosquelette E.

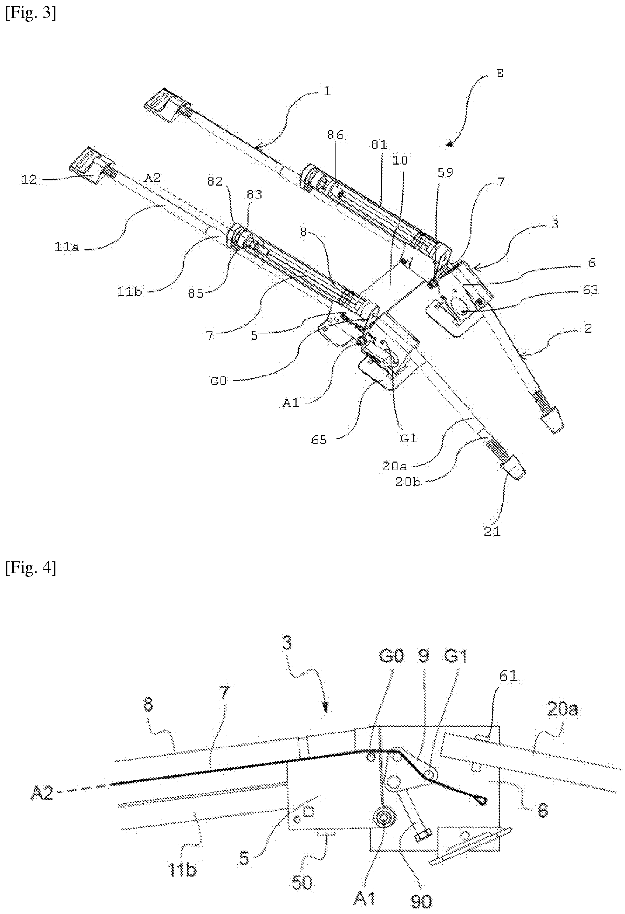

Comme on peut le voir sur les Figures 1 et 3, de manière générale, l'exosquelette E selon la présente invention comprend une structure dorsale 1 axiale, deux structures de liaison de cuisse 2 et un dispositif passif de génération de couple 3 entre la structure dorsale 1 et les structures de liaison de cuisse 2.

Par structure dorsale 1 axiale, on entend une structure dorsale s'étendant le long d'un axe sensiblement parallèle à l'axe A0 de la colonne vertébrale.

La structure dorsale 1 comporte une plaque centrale 10 et deux paires de tubes dorsaux télescopiques 11.

La plaque centrale 10 est une plaque sensiblement rectangulaire destinée à être positionnée, en utilisation, en regard de la région lombaire de l'utilisateur U. Cette plaque 10 comporte des trous traversants pour la liaison au dispositif de génération de couple 3.

Les deux paires de tubes dorsaux télescopiques 11 s'étendent dans un même plan et de manière symétrique par rapport à la plaque centrale 10. Chaque paire de tubes dorsaux 11 comprend un tube supérieur 11a creux et un tube inférieur 11b creux. Le tube supérieur 11a est dimensionné pour être reçu de manière coulissante dans le tube inférieur 11b, les tubes supérieur 11a et inférieur 11b étant reliés entre eux par une liaison pivot glissant. La région d'extrémité libre du tube inférieur 11b est couplée au dispositif de génération de couple 3. La région d'extrémité libre du tube supérieur 11a porte une pièce de fixation 12 à un harnais souple 4, par exemple un adaptateur 12 destiné à recevoir une sangle de bretelle 40. Cet adaptateur 12 peut être relié au tube supérieur 11a par l'intermédiaire d'une pièce mâle sur laquelle le tube 11a est manchonné d'une manière lui permettant au moins de tourner autour de son axe longitudinal. Ainsi, la longueur de la structure dorsale 1, à savoir la distance entre la plaque centrale 10 et la pièce de fixation 12 au harnais 4, peut être adaptée en fonction de 1a corpulence de l'utilisateur U et la liaison pivot glissant permet à l'utilisateur U qui porte la structure dorsale 1 fixée de manière amovible à ses épaules de bouger ses épaules par rapport à son dos et indépendamment l'une de l'autre.

Chaque structure de liaison de cuisse 2 comprend également une paire de tubes de cuisse télescopiques 20. Cette paire 20 comprend un tube supérieur 20a creux et un tube inférieur 20b creux. Le tube inférieur 20b est dimensionné pour être reçu de manière coulissante dans le tube supérieur 20a, les tubes supérieur 20a et inférieur 20b étant reliés entre eux par une liaison pivot glissant. La région d'extrémité libre du tube supérieur 20a est couplée au dispositif de génération de couple 3. La région d'extrémité libre du tube inférieur 20b porte une pièce de fixation 21 au harnais souple 4, par exemple un adaptateur 21 destiné à recevoir une sangle de cuisse 41. Cet adaptateur 21 peut être relié au tube inférieur 20b par l'intermédiaire d'une pièce mâle solidaire de l'adaptateur 21 et sur laquelle le tube 20b est manchonné d'une manière lui permettant de tourner autour de son axe longitudinal. Ainsi, la longueur de chaque structure de liaison de cuisse 2 peut être adaptée en fonction de la taille de l'utilisateur U et la liaison pivot glissant permet à l'utilisateur U qui a les structures de liaison de cuisse 2 fixées de manière amovible à ses cuisses de bouger ses cuisses, indépendamment l'une de l'autre.

En utilisation, la structure dorsale 1 est destinée à être positionnée sur le dos de l'utilisateur U, avec les paires de tubes dorsaux télescopiques 11 sensiblement parallèles à la colonne vertébrale et disposées symétriquement de part et d'autre de celle-ci, chaque paire 11 se trouvant dans le prolongement vertical d'une épaule. Chaque structure de liaison de cuisse 2 est destinée à être positionnée sur la face arrière du corps de l'utilisateur U, entre les fesses et l'une des cuisses. Ainsi, l'exosquelette E est destiné à être positionné uniquement sur la face arrière du corps de l'utilisateur U et ne dépasse pas sur les côtés de l'utilisateur U. Un tel positionnement rend l'exosquelette E commode à utiliser.

Dans le mode de réalisation préféré de la présente invention, le dispositif passif de génération de couple 3 comprend, pour chaque structure de liaison de cuisse 2, un support supérieur 5, un support inférieur 6, un élément de transmission 7 et un organe élastiquement déformable, ici un ressort de compression 8.

On entend par passif le fait que le dispositif de génération de couple 3 est apte à fonctionner sans source d'énergie extérieure, sans capteur et sans motorisation.

Comme on peut le voir sur les Figures 3 à 6, le support supérieur 5 est configuré pour être couplé à la structure dorsale 1 et au ressort 8, pour être articulé en rotation avec le support inférieur 6 et pour être traversé par l'élément de transmission 7 et coopérer avec celui-ci.

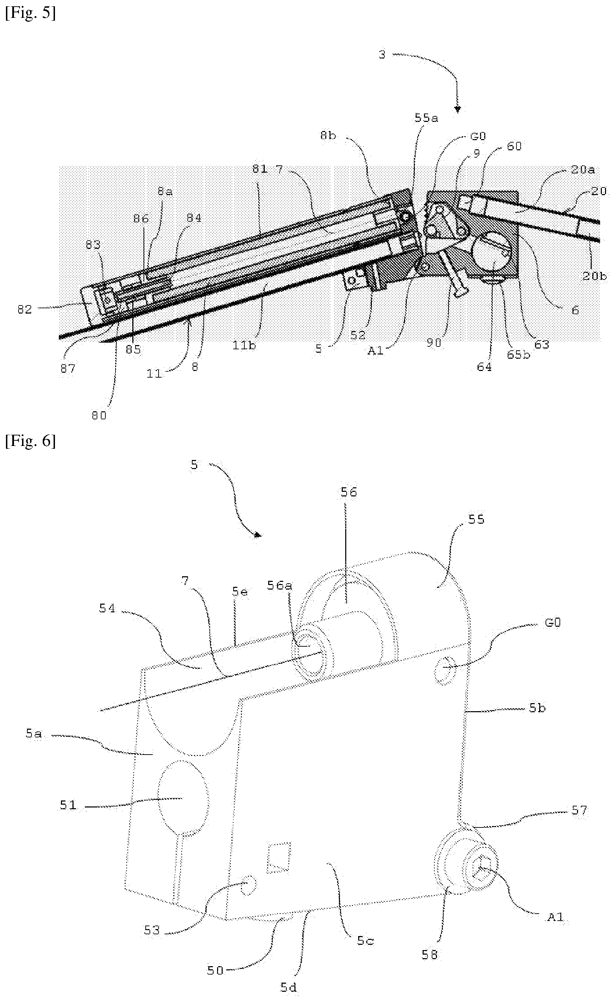

Comme on peut le voir plus en détail sur la [Fig.6], le support supérieur 5 se présente sous la forme d'un bloc de forme générale globalement parallélépipédique ayant deux faces opposées supérieure 5a et inférieure 5b, deux faces latérales 5c opposées et deux faces opposées avant 5d et arrière 5e.

Pour le couplage à la structure dorsale 1, le support supérieur 5 comporte, sur sa face avant 5d, une saillie annulaire 50 apte à être reçue dans le trou traversant correspondant de la plaque centrale 10. Le support supérieur 5 comprend également un alésage 51 traversant le bloc, s'étendant de sa face supérieure 5a à sa face inférieure 5b et débouchant dans la face supérieure 5a. Le diamètre de l'alésage 51 est sensiblement égal au diamètre extérieur du tube dorsal inférieur 11b, de telle sorte que le tube dorsal inférieur 11b est reçu dans l'alésage 51. Pour le maintien en position du tube dorsal inférieur 11b dans l'alésage 51, le support supérieur 5 comporte un trou traversant 52 s'étendant à travers la saillie annulaire 50 et débouchant dans l'alésage 51. Ainsi, un organe de fixation inséré depuis la saillie annulaire 50 vient en appui contre la paroi extérieure du tube dorsal inférieur 11b et permet son maintien radial. En outre, le support supérieur 5 est fendu entre sa région de face avant 5d située entre la saillie annulaire 50 et la face supérieure 5a et l'alésage 51. Un trou traversant 53 s'étend à travers cette zone fendue, d'une face latérale 5c à l'autre. Un organe de fixation, notamment une vis, est reçu dans ce trou traversant 53 et permet de régler l'espacement au niveau de cette zone fendue afin de permettre le blocage du tube dorsal inférieur 11b dans l'alésage 51.

Pour le couplage au ressort 8, le support supérieur 5 comporte, sur sa face arrière 5e, une rainure 54 semi-cylindrique, donc sensiblement en forme de U, ayant une première extrémité ouverte débouchant dans la face supérieure 5a et une seconde extrémité fermée par une paroi circulaire d'un corps cylindrique 55. Le corps cylindrique 55 s'étend de la seconde extrémité de la rainure 54 à la face inférieure 5b. L'axe longitudinal de la rainure 54 est parallèle à l'axe longitudinal de l'alésage 51, lequel axe est légèrement incliné par rapport à un axe perpendiculaire aux faces supérieure 5a et inférieure 5b. La rainure 54 est dimensionnée pour recevoir le ressort 8. Plus particulièrement, une pièce de fixation de ressort 56 est solidaire de la paroi circulaire du corps cylindrique 55. Cette pièce de fixation de ressort 56 présente une tête circulaire appliquée contre la paroi circulaire et une tige cylindrique faisant saillie à partir de la tête et s'étendant coaxialement à la tête. L'une des extrémités du ressort de compression 8, dite extrémité inférieure, est fixée à la tête de la pièce de fixation de ressort 56, et constitue ainsi ladite partie fixe du ressort de compression 8.

Pour le montage en rotation du support inférieur 6 par rapport au support supérieur 5, le support supérieur 5 comporte deux portées cylindriques 57 formées au niveau de l'intersection entre les faces avant 5d et inférieure 5b et faisant saillie par rapport à la face inférieure 5b. Les deux portées 57 sont en regard l'une de l'autre et sont traversées par un alésage traversant dans l'alignement l'un de l'autre. Ces deux alésages reçoivent un axe d'articulation A1 ayant une tige et une tête. La tête vient en appui contre la face de l'une des portées 57 située dans le prolongement de la face latérale 5c, par l'intermédiaire d'une rondelle 58. La tige s'étend à partir de la tête, traverse les deux portées 57 et s'étend au-delà de la portée opposée à celle contre laquelle se trouve la tête. Un écrou 59 et une autre rondelle sont montés sur la portion de la tige en saillie et permettent le maintien en position de l'axe d'articulation A1. Ainsi, l'axe d'articulation A1 est orienté orthogonalement à l'axe de l'alésage 51 recevant le tube dorsal inférieur 11b et orthogonalement à l'axe longitudinal de la rainure 54 et donc du ressort de compression 8.

Pour le passage de l'élément de transmission 7, la tige et la tête de la pièce de fixation de ressort 56 sont traversées par un premier orifice traversant 56a, et le corps cylindrique 55 est traversé par un second orifice traversant 55a dans lequel débouche le premier orifice traversant 56a et débouchant sur la face inférieure 5b. Ainsi, l'élément de transmission 7 traverse le support supérieur 5 au niveau de sa face arrière 5e en s'étendant dans la rainure 54 et en traversant la pièce de fixation de ressort 56 et le corps cylindrique 55.

Pour la coopération du support supérieur 5 avec l'élément de transmission 7, le support supérieur 5 comporte une goupille G0 dont l'axe est parallèle à l'axe d'articulation A1 et s'étendant entre les faces latérales 5c en étant reçue et fixée dans des orifices correspondants prévus au niveau du corps cylindrique 55. Un manchon cylindrique est monté autour de la goupille G0 sensiblement au centre de celle-ci, de façon à se trouver dans le prolongement des premier 56a et second 55a orifices traversants dans lesquels passe l'élément de transmission 7. L'élément de transmission 7 prend appui sur une portion angulaire du manchon, avant de sortir du support supérieur 5 par le second orifice traversant 55a débouchant dans la face inférieure 5b. La goupille G0 constitue donc un premier point d'appui G0 de l'élément de transmission 7 sur le support supérieur 5, avant sa sortie du support supérieur 5. La goupille G0 étant fixée au bloc du support supérieur 5, le premier point d'appui est un point qui est fixe par rapport à l'axe d'articulation A1.

Le support inférieur 6 est configuré pour être couplé à la structure de liaison de cuisse 2 correspondante, pour être monté pivotant par rapport au support supérieur 5 et pour être relié à l'élément de transmission 7.

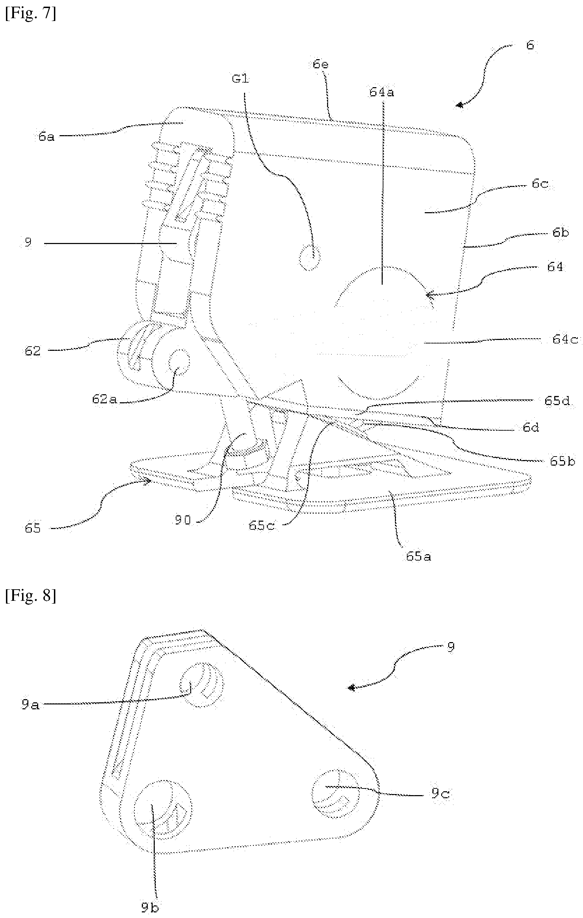

Comme on peut le voir sur la [Fig.7], le support inférieur 6 se présente également sous la forme d'un bloc de forme générale globalement parallélépipédique ayant deux faces opposées supérieure 6a et inférieure 6b, deux faces latérales 6c opposées et deux faces opposées avant 6d et arrière 6e. Le support inférieur 6 se trouve dans un même plan que le support supérieur 5, les deux faces latérales 6c opposées du support inférieur 6 étant sensiblement dans le prolongement des deux faces latérales 5c opposées du support supérieur 5.

Pour le couplage à la structure de liaison de cuisse 2, le support inférieur 6 comprend un alésage 60 ayant une extrémité ouverte débouchant dans la face inférieure 6b et une extrémité fermée située à l'intérieur du bloc. Le diamètre de l'alésage 60 est sensiblement égal au diamètre extérieur du tube de cuisse supérieur 20a, de telle sorte que le tube de cuisse supérieur 20a est reçu dans l'alésage 60. L'alésage 60 est fendu sur sensiblement toute sa longueur, laquelle fente débouche dans l'une des faces latérales 6c. Pour le maintien en position du tube de cuisse supérieur 20a dans l'alésage 60, le support inférieur 6 comporte un trou traversant 61 s'étendant depuis sa face arrière 6e, débouchant dans la fente et se prolongeant dans le bloc. Ainsi, un organe de fixation, notamment une vis, est reçu dans ce trou traversant 61 et permet de régler l'espacement entre les parois de la fente afin de permettre le blocage du tube de cuisse 20a dans l'alésage 60.

Pour le montage pivotant du support inférieur 6 par rapport au support supérieur 5, le support inférieur 6 comporte une portée cylindrique 62 formée au niveau de l'intersection entre les faces supérieure 6a et avant 6d et faisant saillie par rapport à la face supérieure 6a. La portée 62 est positionnée sensiblement au centre de la face supérieure 6a du bloc et est dimensionnée de manière à venir s'interposer entre les deux portées cylindriques 57 du support supérieur 5. La portée 62 est traversée par un alésage traversant 62a, agencé pour se trouver dans l'alignement des alésages des deux portées cylindriques 57 du support supérieur 5. A l'état monté, cet alésage 62a reçoit l'axe d'articulation A1. Ainsi, l'axe d'articulation A1 traverse les deux portées cylindriques 57 du support supérieur 5 et la portée cylindrique 62 du support inférieur 6. L'axe d'articulation A1 étant fixé aux portées cylindriques 57 du support supérieur 5, c'est la portée cylindrique 62 du support inférieur 6 qui est apte à tourner par rapport à l'axe d'articulation A1. Ainsi, en utilisation, le support inférieur 6 est apte à tourner autour de l'axe d'articulation A1 de manière à effectuer un mouvement de pivotement dans un plan perpendiculaire à l'axe d'articulation A1.

Pour la liaison de l'élément de transmission 7 au support inférieur 6, le support inférieur 6 comporte une goupille cylindrique 63 autour de laquelle s'enroule la région de l'une des extrémités libres de l'élément de transmission 7 et à laquelle est fixée cette extrémité libre. Cette goupille 63 constitue ainsi le premier point de fixation de l'élément de transmission 7. La goupille cylindrique 63 s'étend parallèlement à l'axe d'articulation A1, entre les deux faces latérales 6c opposées et au voisinage de la face inférieure 6b.

De manière avantageuse, la goupille cylindrique 63 est solidaire d'un tambour d'enroulement 64 qui permet, au besoin, d'éviter la transmission du déplacement de l'élément de transmission 7 au ressort 8. Le tambour d'enroulement 64 comporte un moyeu cylindrique 64a monté tournant dans un alésage correspondant prévu dans le support inférieur 6. L'axe longitudinal du moyeu cylindrique 64a s'étend parallèlement à l'axe de la goupille cylindrique 63. Sur sa périphérie, le moyeu 64a comporte deux gorges, espacées l'une de l'autre, et parcourant sensiblement la moitié de la circonférence du moyeu 64a. Ces deux gorges s'étendent dans des plans parallèles entre eux et parallèles au plan du support inférieur 6, donc perpendiculaire à l'axe de la goupille cylindrique 63. Deux axes (non visibles), solidaires de la face avant 6d du support inférieur 6 sont reçus dans chacune des deux gorges. Ces axes permettant ainsi de guider la rotation du moyeu 64a dans l'alésage et de définir sa plage angulaire de rotation, limitée par les extrémités des gorges, qui forment alors des butées en rotation. La goupille cylindrique 63 est solidaire du moyeu 64a et traverse ce moyeu 64a. Le tambour d'enroulement 64 comporte également une poignée 64c, solidaire de l'une des bases du moyeu cylindrique 64a, en particulier la base destinée à être tournée vers l'extérieur, en utilisation. Cette poignée 64c peut donc être manipulée par l'utilisateur U afin de commander le mouvement de rotation du tambour d'enroulement 64. Ainsi, le tambour 64 est monté mobile en rotation entre deux positions, à savoir une position embrayée et une position débrayée. Plus précisément, une rotation de la poignée 64c dans un sens de rotation ou dans l'autre entraîne une rotation également dans ce sens de rotation du moyeu 64a et de la goupille cylindrique 63. Cette rotation de la goupille cylindrique 63 provoque, selon le sens de rotation, soit l'enroulement de l'élément de transmission 7 autour de la goupille cylindrique 63 soit son déroulement. Lorsque l'élément de transmission 7 est enroulé, sa longueur diminue, jusqu'à une position d'enroulement maximal, dite position embrayée, dans laquelle une rotation du support inférieur 6 par rapport au support supérieur 5 provoquera un déplacement de l'élément de transmission 7 qui entraîne une compression du ressort 8. Au contraire, lorsque l'élément de transmission 7 est déroulé par rapport à la goupille cylindrique 63, sa longueur augmente, jusqu'à une position de déroulement maximal, dite position débrayée, dans laquelle une rotation du support inférieur 6 par rapport au support supérieur 5 ne soumet pas l'élément de transmission 7 à une force de traction et ne provoque donc pas de compression du ressort 8. On pourra placer le tambour d'enroulement 64 dans la position débrayée si besoin lorsque l'utilisateur U enfile l'exosquelette 1.

Ce tambour 64 constitue un module de mise en traction/détente de l'élément de transmission 7, qui dans ce mode de réalisation particulier de l'invention, est un module à commande manuelle. Ce module de mise en traction/détente de l'élément de transmission 7 a ainsi pour fonction de placer l'élément de transmission 7 dans un état dans lequel il est mis en traction ou dans un état dans lequel il est détendu, par le réglage de la longueur de l'élément de transmission 7 entre ses deux points de fixation.

Le support inférieur 6 est également configuré pour permettre un réglage du couple de redressement. Pour cela, le support inférieur 6 comporte une fourchette 9 de réglage de couple. La fourchette 9 est positionnée à l'intérieur du support inférieur 6, entre sa face supérieure 6a ouverte, depuis laquelle elle est accessible, et le tambour d'enroulement 64. Comme on peut le voir sur la [Fig.8], la fourchette de réglage de couple 9 se présente sous la forme d'une pièce globalement triangulaire ayant un orifice traversant au niveau de chacun de ses trois coins, à savoir un orifice arrière 9a, un orifice avant supérieur 9b et un orifice avant inférieur 9c. L'axe de chaque orifice 9a, 9b, 9c est parallèle à l'axe d'articulation A1. Une goupille est reçue dans chacun de ces orifices, à savoir une goupille arrière, une goupille avant supérieure et une goupille avant inférieure G1. Cette goupille G1 constitue le second point d'appui, entre l'élément de transmission 7 et le support inférieur 6. La fourchette 9 a également des fentes au niveau de ses trois coins. En particulier, deux fentes allongées sont formées dans la face avant de la fourchette et débouchent dans les orifices avant 9b, 9c. Une troisième fente, sensiblement triangulaire, communique avec l'orifice arrière 9a. Ces fentes permettent le réglage de la position de la fourchette 9 à l'intérieur du support inférieur 6, et donc le réglage de la position de la goupille avant inférieure G1 par rapport à l'axe d'articulation A1. En particulier, une vis 90, introduite depuis la face avant 6d du support inférieur 6 est reçue dans la fente avant supérieure et permet de positionner la fourchette 9 à la position souhaitée. La fourchette 9 comporte également, à l'intérieur de celle-ci, un chemin formé entre la fente communiquant avec l'orifice arrière 9a et la fente débouchant dans l'orifice avant inférieur 9c.

En outre, le support inférieur 6 porte un appui-fesses 65 destiné à venir se positionner contre la fesse correspondante de l'utilisateur U. L'appui-fesses 65 comporte une plaque rectangulaire 65a reliée à la face avant 6d du support inférieur 6. En particulier, l'appui-fesses 65 est relié au support inférieur 6 par une liaison rotule. La liaison rotule est formée par une portion de sphère 65b située dans un évidement 65c de forme complémentaire à celle de la portion de sphère 65b. La portion de sphère 65b fait saillie à partir d'une plaque 65d solidaire de la face avant 6d du support inférieur 6. L'évidement 65c est formé dans une partie inclinée par rapport à la plaque 65a d'appui-fesses et solidaire de celle-ci.

Le ressort de compression 8 est agencé et configuré pour produire, en utilisation, une force de rappel F rectiligne le long d'un axe de déformation A2 orthogonal à l'axe d'articulation A1 et dont la grandeur est linéairement proportionnelle à sa déformation élastique.

Le ressort de compression 8 est un ressort de compression cylindrique comprenant une pluralité de spires agencées autour d'un axe de déformation A2 globalement parallèle à l'axe des tubes dorsaux 11. Le ressort 8 a une extrémité supérieure 8a, qui constitue une partie mobile, et une extrémité inférieure 8b opposée, qui comme indiqué ci-dessus constitue la partie fixe. L'extrémité inférieure 8b est ainsi solidaire de la pièce de fixation de ressort 56. La région d'extrémité du ressort 8 située côté extrémité inférieure 8b s'étend le long de la rainure 54 formée sur le support supérieur 5. L'extrémité supérieure 8a est solidaire d'une autre pièce de fixation de ressort 80 similaire.

Le ressort 8 est entouré par un fourreau 81 sous la forme d'un tube cylindrique de diamètre sensiblement supérieur à celui du ressort 8. Ainsi, le fourreau 81 ne gêne pas le déplacement du ressort 8 et empêche tout contact de l'utilisateur U avec les spires du ressort 8, assurant ainsi une sécurité de fonctionnement. Ce tube est réalisé en une matière plastique transparente, par exemple en PMMA. Le fourreau 81 s'étend coaxialement au ressort 8 entre le corps cylindrique 55 et un embout 82 solidaire du tube dorsal inférieur 11b. Ainsi, le fourreau 81 est relié au tube dorsal 11b par l'intermédiaire de l'embout 82 et au support supérieur 5.

Entre l'embout 82 et l'extrémité supérieure 8a du ressort 8 se trouve une pièce de fixation 83 d'un tendeur 84 permettant de régler la tension à vide de l'élément de transmission 7, notamment pour laisser un mou permettant d'éviter l'actionnement du ressort 8 lorsque l'utilisateur U marche. Le tendeur 84 peut être un simple tendeur classique, à écrous 85, dont une première extrémité est fixée à la pièce de fixation 83, par exemple par l'intermédiaire d'une goupille transversale, et dont une seconde extrémité est fixée à la seconde extrémité de l'élément de transmission 7 et porte un élément d'appui 86 en appui sur l'extrémité du ressort 8.

Il convient de souligner que les ressorts de compression 8 pourraient être remplacés par tout organe élastiquement déformable apte à produire une force rectiligne F le long d'un axe de déformation orthogonal à l'axe d'articulation A1 et dont la grandeur est linéairement proportionnelle à sa déformation élastique.

En variante, le dispositif passif de génération de couple 3 pourrait également comprendre un unique ressort de compression pour les deux structures de liaison de cuisse 2. Dans cette variante (non représentée), l'unique ressort de compression est couplé à chacun des deux supports supérieurs 5 par l'intermédiaire d'un système de poulies analogue au système de poulies décrit dans la demande de brevet français FR3110476 et coopérant avec les éléments de transmission 7. Plus précisément, dans cette variante, l'unique ressort de compression s'étend selon un axe de déformation parallèle à l'axe de la structure dorsale 1 et orthogonal à l'axe d'articulation A1. Les extrémités supérieures des deux éléments de transmission 7 sont réunies en un point central et ils sont fixés à l'unique ressort. Le système de poulies comprend deux poulies intermédiaires positionnées au niveau de l'extrémité inférieure du ressort et renvoyant les éléments de transmission 7 de part au d'autre vers les supports supérieurs 5. Chaque élément de transmission 7 attaque alors sensiblement verticalement de chaque côté une poulie d'extrémité, jusqu'à son point de fixation 63 au support inférieur 6.

Dans le mode de réalisation préféré, l'élément de transmission 7 est agencé et configuré pour provoquer, en utilisation, la compression du ressort 8 lors d'un pivotement relatif entre le support supérieur 5 et le support inférieur 6 et pour transmettre la force de rappel F générée par le déplacement du ressort 8 afin de délivrer un couple de redressement C autour de l'axe d'articulation A1. Comme indiqué ci-dessus, en raison du mou prévu dans l'élément de transmission 7, le ressort 8 ne sera pas comprimé lorsque l'utilisateur marche.

L'élément de transmission 7 se présente sous la forme d'un câble réalisé en un matériau souple et inextensible. De préférence, le câble est en fibres de polyéthylène haut module, de type HPME ou UHMwPE, par exemple commercialisées sous la dénomination Dyneema^{®}.

Le câble 7 a une première extrémité fixée à la goupille cylindrique 63 solidaire du tambour d'enroulement 64, laquelle goupille 63 constitue un premier point de fixation, et une seconde extrémité opposée fixée au tendeur 84 en un second point de fixation 87. Le câble 7 est guidé entre ses deux points de fixation 63, 87 afin de traverser successivement le support inférieur 6, le support supérieur 5 et le ressort 8. En particulier, le câble 7 est guidé par la fourchette 9 après avoir quitté le tambour d'enroulement 64. Il vient en appui contre la goupille avant inférieure G1, côté face avant 6d, puis en appui contre la goupille arrière, côté face arrière 6e. Le câble 7 sort ensuite du support inférieur 6 après avoir traversé la fente triangulaire, puis entre dans le support supérieur 5 par le second orifice traversant 55a du corps cylindrique 55. Ensuite, le câble 7 passe autour du manchon cylindrique entourant la goupille G0 solidaire du support supérieur 5. Il traverse ensuite les deux pièces de fixation de ressort 56 et 80, en passant à l'intérieur du ressort 8 et en s'étendant le long de l'axe de déformation A2 du ressort 8. Enfin, le câble 7 est fixé au tendeur 84. Ainsi, l'élément de transmission 7 suit un premier chemin rectiligne entre son premier point de fixation 63 et la goupille avant inférieure G1 de la fourchette 9 qui constitue son second point d'appui, avec le support inférieur 6, un deuxième chemin rectiligne entre ce point d'appui et la goupille arrière de la fourchette 9, un troisième chemin rectiligne entre la goupille arrière de la fourchette 9 et la goupille G0 constituant son premier point d'appui, avec le support supérieur 5, et un quatrième chemin rectiligne entre ce point d'appui et son second point de fixation 87.

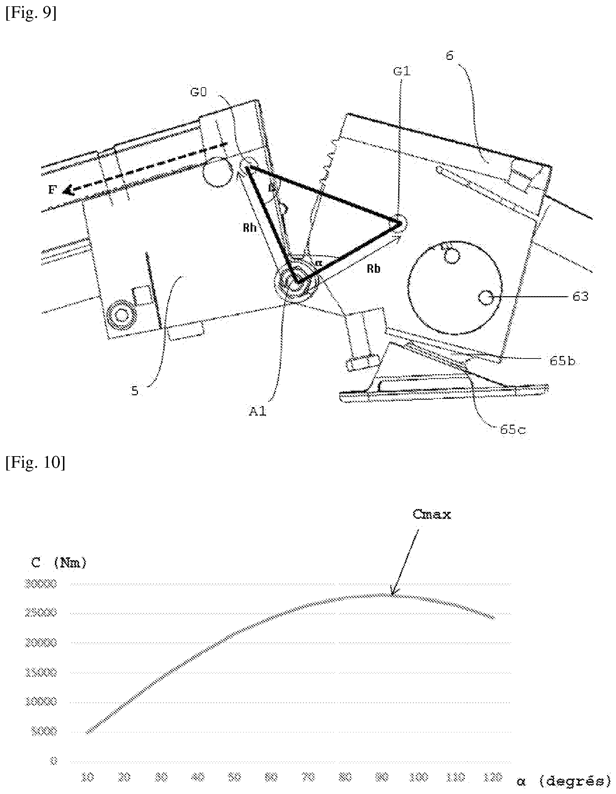

La distance entre l'axe de la goupille G0 constituant le premier point d'appui et l'axe d'articulation A1 est une distance fixe Rh ([Fig.9]). De même, une fois la position de la fourchette de réglage de couple 9 réglée, la distance entre l'axe de la goupille avant inférieure G1 de la fourchette 9 et l'axe d'articulation A1 est une distance fixe Rb. Ainsi, l'axe d'articulation A1, l'axe passant par le premier point d'appui G0 et l'axe passant par le second point d'appui G1 définissent les sommets d'un triangle, comme représenté sur la [Fig.9], lequel triangle est déformable par le changement de la position de 1a fourchette 9 et par le pivotement relatif entre les supports supérieur 5 et inférieur 6 autour de l'axe d'articulation A1.

L'exosquelette E selon la présente invention est destiné à être porté par un utilisateur U par l'intermédiaire d'un harnais souple 4 ou harnais textile relié à la structure dorsale 1 et à chaque structure de liaison de cuisse 2.

Comme on peut le voir sur la [Fig.1], ce harnais souple 4 comporte des bretelles d'épaule 42, des sangles de cuisse 41, une ceinture 43 et des coussins d'interface 44, 45.

Les bretelles 42 sont reliées entre elles par une partie de dossier destinée à venir en contact avec le haut du dos de l'utilisateur U. Les bretelles 42 portent chacune une attache de sangle. Une sangle de bretelle 40 relie chaque attache de sangle avec la pièce de fixation 12 correspondante portée par chaque tube supérieur 11a de la structure dorsale 1. Les bretelles 42 sont également reliées à la ceinture 43 par l'intermédiaire d'attaches de sangle et de sangles.

La ceinture 43 relie la structure dorsale 1 à l'utilisateur U au niveau de la plaque centrale 10.

Les sangles de cuisse 41 sont reçues dans les pièces de fixation 21 portées par chaque tube inférieur de cuisse 20b. Ces sangles 41 sont destinées à entourer la cuisse de l'utilisateur U.

Chacune des sangles 40, 41 est réglable en longueur afin de permettre un réglage du harnais 4 en fonction de la corpulence de l'utilisateur U.

Les coussins d'interface 44, 45 permettent d'assurer le confort de l'utilisateur U. De préférence, le harnais 4 comprend des coussins de cuisse 44 et un coussin de fesses 45. Les coussins de cuisse 44 sont agencés pour être interposés entre les sangles de cuisse 41 et les cuisses de l'utilisateur U, en utilisation. Le coussin de fesses 45 est agencé pour être interposé entre les appui-fesses 65 et les fesses de l'utilisateur U, en utilisation.

Un tel harnais souple 4 est léger, facile à enfiler et facilement réglable.

En utilisation, une fois le harnais 4 enfilé par l'utilisateur U, lorsque l'utilisateur U est en posture debout, avec l'angle entre son tronc et ses cuisses compris entre environ 175 degrés et 185 degrés, pour chaque structure de liaison de cuisse 2, les supports supérieur 5 et inférieur 6 associés sont en contact entre eux, l'élément de transmission 7 n'est pas soumis à une force de traction et le ressort 8 n'est pas déformé élastiquement, autrement dit il n'est pas comprimé. De ce fait, aucun couple de redressement C n'est appliqué par le dispositif de génération de couple 3. Il en va de même lors de la marche.

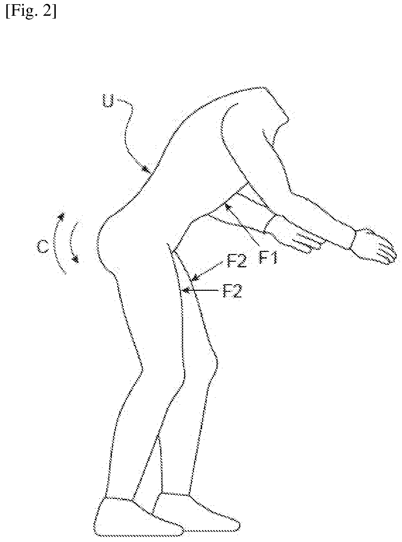

Lorsque l'utilisateur U se penche en avant et que l'angle entre son dos et l'arrière de ses cuisses augmente, chaque support inférieur 6 pivote par rapport au support supérieur 5 associé autour de l'axe d'articulation A1. L'angle formé entre les faces avant 5d, 6d des supports supérieur 5 et inférieur 6 correspond à l'angle entre le dos et l'arrière des cuisses de l'utilisateur U. Une fois les tambours d'enroulement 64 placés en position embrayée, chaque élément de transmission 7 est soumis à une force de traction sous l'action de l'inclinaison de l'utilisateur U. Chaque élément de transmission 7 amène alors le ressort 8 associé à se comprimer, autrement dit le ressort 8 est déformé de sorte que son extrémité supérieure 8a se rapproche de son extrémité inférieure 8b. Ce déplacement amène chaque ressort 8 à appliquer une force de rappel F, exprimée en Newtons (N), qui est exprimée par la formule F = k x ΔL, où k est la constante de rappel du ressort 8 exprimée en Newtons par mètre (N/m) et ΔL sa déformation exprimée en mètres (m). La constante de rappel k de chaque ressort 8 peut par exemple être de 17,6 N/mm. Cette force de rappel F permet de générer, par l'intermédiaire de l'élément de transmission 7, un couple de redressement C autour de l'axe d'articulation A1, donc entre la structure dorsale 1 et chaque structure de liaison de cuisse 2. Ce couple de redressement C amène alors la structure dorsale 1 à appliquer une force de résistance F1 sur le tronc de l'utilisateur U et les structures de liaison de cuisse 2 à appliquer une force de résistance F2 sur les cuisses de l'utilisateur U, lesquelles forces de résistance sont orthogonales au tronc et aux cuisses, respectivement ([Fig.2]). De telles forces F1, F2 étant orthogonales au corps de l'utilisateur U, elles peuvent être importantes, contrairement à des forces tangentielles qui sont moins facilement tolérées.

En raison de la configuration du dispositif de génération de couple 3, en particulier en raison de l'agencement de l'élément de transmission 7 par rapport à l'axe d'articulation A1, bien que la force de rappel F de chaque ressort 8 augmente de manière linéairement proportionnelle à l'augmentation de la déformation ΔL, le couple de redressement C généré ne suit pas une loi linéaire, mais une loi sinusoïdale ou pseudo-sinusoïdale. En effet, comme représenté sur la [Fig.10], le vecteur force est appliqué sur l'élément de transmission 7 dans une direction différente de celle de la force de rappel F puisque l'élément de transmission 7 s'étend dans la direction du ressort 8 entre son second point de fixation 87 et son premier point d'appui G0 contre le support supérieur 5, mais change de direction, au moins une fois, entre son premier point d'appui G0 et son point d'appui G1 contre le support inférieur 6. Ainsi, le couple de redressement C appliqué autour de l'axe d'articulation A1 s'exprime par la formule C = A x sin(β).

A, qui s'exprime en Newton mètre (Nm), correspond au produit de la valeur de la force appliquée sur l'élément de transmission 7, donc la valeur de la force de rappel F, par la longueur du vecteur position Rh, à savoir A = F x Rh. Rh est une constante connue qui correspond à la distance (en mètres) entre le point de rotation (axe d'articulation A1) et le point d'application de la force, à savoir le premier point d'appui G0. Autrement dit, Rh est la longueur de l'un des côtés du triangle déformable comme représenté sur la [Fig.10].

L'angle β représente l'angle (en radians) formé entre le vecteur force et le vecteur position, donc l'angle formé entre deux côtés du triangle déformable, à savoir entre le côté de longueur Rh et un côté du triangle qui s'étend entre le premier point d'appui G0 et le second point d'appui G1. Cet angle β dépend de la longueur Rb du côté du triangle opposé à l'angle β, autrement dit le côté du triangle qui s'étend entre le sommet définit par l'axe d'articulation A1 et le sommet définit par le second point d'appui G1, de la longueur Rh et de l'angle α formé entre les côtés du triangle de longueurs Rh et Rb. Cet angle α est directement proportionnel à l'angle d'inclinaison entre le dos et l'arrière des cuisses de l'utilisateur U. Plus exactement, l'angle β s'exprime comme suit : β = arctan [(sin(α) x Rb)/(Rh - Rb x cos(α))].

En conséquence, la force de résistance F1, respectivement F2, appliquée par la structure dorsale 1 sur le tronc de l'utilisateur U, respectivement sa cuisse, est égale au rapport du couple de redressement C à la distance entre l'axe d'articulation A1 et le point d'application de la force de résistance F1 sur le tronc, respectivement de la force de résistance F2 sur la cuisse, lesquelles distances peuvent être considérées comme fixes lors de l'utilisation.

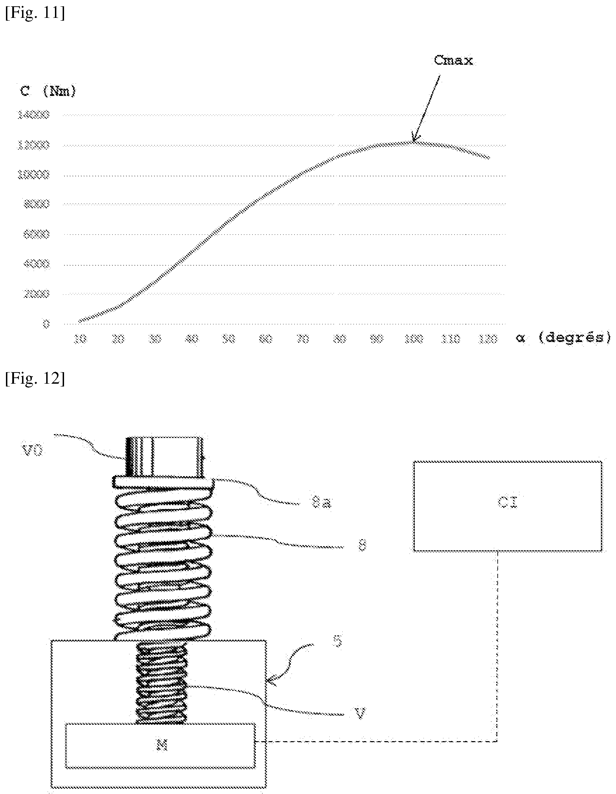

Les Figures 10 et 11 montrent la valeur du couple de redressement C en fonction de l'angle α, le couple de redressement C étant calculé selon les formules exprimées ci-dessus.

Par exemple, comme représenté sur la [Fig.10], pour une longueur Rh de 40 mm, une longueur Rb réglée à 40 mm et une constante de rappel k de chaque ressort 8 de 17,6 N/mm, le couple de redressement maximal Cmax est obtenu pour un angle α d'environ 90 degrés et a une valeur d'environ 28000 Nm. Lorsque l'angle α dépasse cet angle d'environ 90 degrés, le couple de redressement C diminue.

Comme on peut le voir sur la [Fig.11], pour une longueur Rh de 40 mm, une longueur Rb réglée à 25 mm par la modification de la position de la fourchette 9, donc inférieure à la longueur de Rb sur la courbe de la [Fig.11], et une constante de rappel k de chaque ressort 8 de 17,6 N/mm, le couple de redressement maximal Cmax est obtenu pour un angle α d'environ 100 degrés et a une valeur d'environ 12000 Nm. Ainsi, la diminution de la longueur Rb permet d'augmenter l'angle α pour lequel le couple de redressement maximal Cmax est appliqué et de diminuer la valeur de ce couple de redressement Cmax. Ceci permet également de diminuer la valeur du couple de redressement maximal Cmax qui est appliqué pour des angles α qui sont faibles, en particulier, inférieurs à 20 degrés. Un tel réglage peut donc s'avérer utile pour un utilisateur U de faible corpulence qui aura plus de mal à vaincre la résistance appliquée lorsqu'il commence à se pencher qu'un utilisateur ayant une plus grande corpulence.

Ainsi, un utilisateur U qui porte l'exosquelette E selon la présente invention, configuré et réglé pour appliquer le couple résistant C tel que représenté sur l'une quelconque des Figures 11 et 12, se verra appliquer des forces de résistance F1, F2 peu importantes lorsqu'il commencera à se pencher en avant. Puis, il sera soutenu par l'exosquelette E lorsqu'il se trouvera dans une posture penchée en avant dans laquelle son dos subirait un moment de flexion important en l'absence d'exosquelette E. Toutefois, en raison de la loi sinusoïdale suivie par le couple résistant C, l'utilisateur U parviendra à se baisser jusqu'au sol, sans être entravé par les forces de résistance F1, F2 qu'il subit, celui-ci diminuant au-delà d'un certain angle d'inclinaison. L'exosquelette E selon la présente invention est donc particulièrement adapté à un travailleur qui doit rester en posture penchée en avant de manière prolongée, mais qui doit également pouvoir marcher ou se baisser jusqu'au sol.

L'exosquelette E décrit ci-dessus est un exosquelette ne comportant aucune motorisation.

L'on pourrait toutefois prévoir une commande automatique du tambour d'enroulement, dont la rotation serait entraînée par un servomoteur, lui-même commandé par exemple simplement par un bouton actionnable par l'utilisateur, qui n'a alors plus à faire tourner lui-même le tambour d'enroulement pour l'embrayer ou le débrayer.

On pourrait de plus prévoir une centrale inertielle configurée, par exemple, pour détecter, d'une manière bien connue en soi, lorsque l'utilisateur va s'asseoir sans incliner son buste, de façon à commander automatiquement le servomoteur pour débrayer le tambour d'enroulement. La centrale inertielle pourrait également être remplacée par divers capteurs reliés à un contrôleur et aptes à fournir diverses informations, telles que l'angle d'inclinaison du buste de l'utilisateur U, l'angle du buste par rapport aux cuisses, ou encore un capteur de force ou de pression.

Selon une variante du mode de réalisation décrit ci-dessus, l'exosquelette E peut comprendre un module de pilotage du couple de redressement qui peut être actionné à des instants choisis par l'utilisateur U ou en fonction de paramètres programmés. Par exemple, comme représenté schématiquement sur la [Fig.12], ce module de pilotage pourrait comprendre pour chaque structure de liaison de cuisse 2, un actionneur motorisé, ici un moteur électrique M rotatif, monté sur le support supérieur 5 et configuré pour déplacer en translation une vis d'actionnement V disposée coaxialement au ressort 8. La vis d'actionnement V peut être reliée à l'extrémité supérieure de l'élément de transmission 7 ou couplée à l'extrémité supérieure 8a du ressort 8, à laquelle l'élément de transmission 7 reste fixé. La vis d'actionnement V comporte un alésage longitudinal à travers lequel passe l'élément de transmission 7. Dans le cas où la vis d'actionnement V est couplée au ressort 8, un écrou V0 est monté sur la vis V de telle sorte que le ressort 8 se trouve entre un appui fixe du support supérieur 5 et l'écrou mobile V0. Le moteur M pourra être placé dans toute position permettant le cheminement de l'élément de transmission 7 comme décrit pour le mode de réalisation illustré sur les Figures 1 à 9.

Ce module de pilotage fonctionne de la manière suivante. Pour comprimer le ressort 8, afin que la compression du ressort 8 soit supérieure à celle qui serait provoquée sous la seule action de l'utilisateur U, on déclenche le moteur électrique M dans le sens qui déplace la vis V en translation dans le sens de la compression du ressort 8. De ce fait, l'écrou V0 est entraîné en translation par la vis V et appui contre l'extrémité supérieure 8a du ressort, ce qui comprime le ressort 8 indépendamment de la posture de l'utilisateur U. Lorsque le ressort 8 est comprimé par l'écrou V0, le ressort 8 est empêché de se détendre, par exemple par un frein à manque de courant interposé entre le moteur électrique M et la vis V. Lorsque le moteur M est actionné dans le sens qui déplace la vis V en translation dans le sens de la détente du ressort 8, l'écrou V0 est entraîné en translation dans ce même sens de déplacement et permet alors la détente du ressort 8 et donc le pilotage de la force de rappel F générée par ce ressort 8. Comme indiqué ci-dessus, chaque moteur M pourrait être commandé en fonction de moyens de détermination de l'angle d'inclinaison du buste de l'utilisateur U, par exemple une centrale inertielle CI, bien connus en soi et agencés pour détecter l'angle d'inclinaison du buste par rapport à chaque cuisse et reliés à un contrôleur.

Un autre exemple de mise en œuvre d'un tel module de pilotage est le tambour d'enroulement 64, dans sa version motorisée, puisqu'il permet de tirer sur l'élément de transmission 7 par simple enroulement de celui-ci sur le tambour d'enroulement 64.

Un tel module de pilotage a donc pour fonction de fournir au dispositif de génération de couple 3 l'énergie nécessaire à la génération d'un couple de redressement C, à la place de l'utilisateur U. De plus, grâce à un tel module de pilotage, il est possible d'appliquer une force de traction sur l'élément de transmission 7 ou au contraire de le détendre, ou en variante, d'appliquer une force de compression sur le ressort 8 ou au contraire de le détendre, indépendamment de l'angle α, et donc d'entraîner la génération d'un couple de redressement C, uniquement lorsque le moteur M est commandé de manière à soumettre l'élément de transmission 7 à une force de traction ou de manière à comprimer le ressort 8, et donc sans que l'utilisateur U n'ait à fournir l'énergie nécessaire pour générer une force de rappel F. Ainsi, ce module de pilotage permet d'envisager un pilotage de la force de traction appliquée sur l'élément de transmission 7 permettant, par exemple, de décaler le couple de redressement maximal Cmax à un angle d'inclinaison α de 45 degrés, par la commande de la compression du ressort, α étant l'angle formé entre les segments de longueur Rh et Rb, et d'effacer le couple de redressement C au-delà de cet angle d'inclinaison, par la commande de la détente du ressort 8 afin de ne pas augmenter, voire d'annuler, la force de rappel F générée.

Ainsi, l'exosquelette E selon la présente invention est apte à générer un couple de redressement à partir de la seule énergie mécanique fournie par l'utilisateur, lequel couple suit alors une loi pseudo-sinusoïdale en fonction de l'angle d'inclinaison entre le dos et les cuisses de l'utilisateur, et peut également être commandé, lorsque l'utilisateur le souhaite, par un module de pilotage apte à fournir l'énergie nécessaire à la génération d'un couple de redressement indépendamment de l'angle d'inclinaison entre le dos et les cuisses de l'utilisateur.

Dans le premier mode de réalisation décrit ci-dessus, les supports supérieur 5 et inférieur 6 sont chacun un ensemble rigide, c'est-à-dire non articulé.

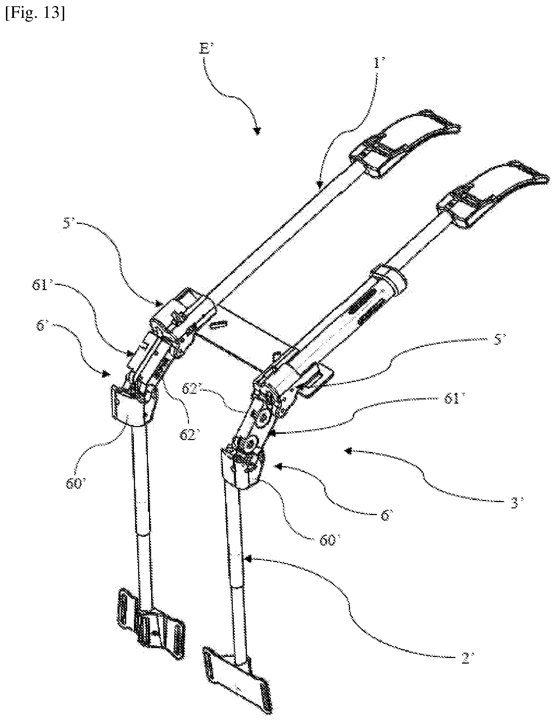

Si l'on se réfère maintenant aux Figures 13 à 17, on peut voir que l'on y a représenté un exosquelette E' selon un second mode de réalisation de la présente invention, dont le dispositif de génération de couple 3' comprend des supports supérieurs 5' qui sont rigides et des supports inférieurs 6' qui sont articulés et comprennent chacun un corps de base 60' et un ensemble d'articulation à maillon(s) 61' qui agit à la manière d'une ou plusieurs vertèbres, selon qu'il comprend un ou plusieurs maillons rigides 62', 62'a, 62'b. On souligne que les autres caractéristiques décrites ci-dessus pour le premier mode de réalisation peuvent être appliquées au second mode de réalisation.

Dans l'exemple illustré sur les Figures 13 à 16, l'ensemble d'articulation à maillon(s) 61' comprend un seul maillon 62'.

Le corps de base 60' est une pièce rigide qui est couplée à la structure de liaison de cuisse 2' de manière analogue au premier mode de réalisation, et le support supérieur 5' est couplé à la structure dorsale 1' également de manière analogue au premier mode de réalisation.

Le maillon 62' est une pièce rigide dont une première extrémité est articulée en rotation au support supérieur 5' autour de l'axe d'articulation A1 et dont la seconde extrémité est articulée au corps de base 60' autour d'un axe d'articulation secondaire A3 qui est parallèle à l'axe d'articulation A1.

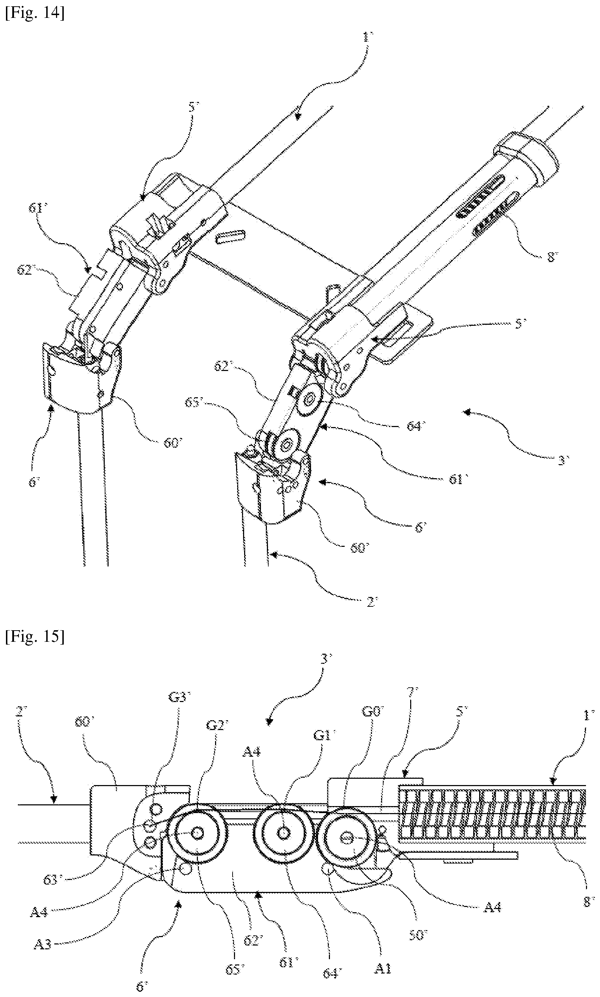

Le support supérieur 5', le corps de base 60' et le maillon 62' sont ici agencés pour qu'au repos, à savoir lorsque l'utilisateur U se tient droit à la verticale, ils soient alignés les uns avec les autres et pour que l'axe d'articulation A1 et l'axe d'articulation secondaire A3 appartiennent à un plan vertical, comme on peut le voir sur la [Fig.15].

Le support supérieur 5' porte ici une première poulie 50' au voisinage de son extrémité articulée au maillon 62', laquelle première poulie 50' constitue le premier point d'appui pour l'élément de transmission 7'. Le maillon 62' porte des deuxième et troisième poulies 64', 65' au voisinage de sa première et de sa seconde extrémité, respectivement. Les première à troisième poulies 50', 64', 65' sont toutes montées à rotation libre autour d'axes de rotation A4 parallèles à l'axe d'articulation A1.

L'élément de transmission 7', dont la seconde extrémité est reliée à une partie mobile de l'organe élastiquement déformable 8' au niveau du second point de fixation, est amené à passer sur les première à troisièmes poulies 50', 64', 65' jusqu'à atteindre le corps de base 60' où il est fixé au niveau du premier point de fixation 63'. Chacune des première à troisièmes poulies 50', 64', 65' constituent donc un point d'appui pour l'élément de transmission 7' lorsque l'utilisateur U se penche en avant.

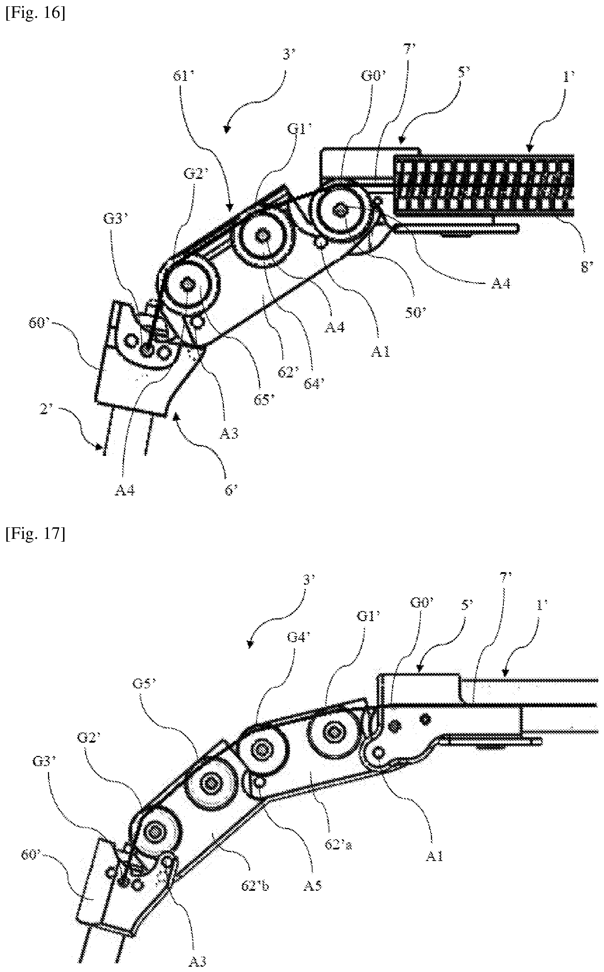

Dans ce second mode de réalisation, le premier point d'appui G0', correspondant au premier point d'appui G0 du premier mode de réalisation, est formé par la première poulie 50', l'élément de transmission 7' prenant appui sur une portion angulaire de la surface circonférentielle de la première poulie 50'. De manière analogue, le second point d'appui G1', correspondant au second point d'appui G1 du premier mode de réalisation, est formé par la deuxième poulie 64' portée par le maillon 62'. Les positions des première et deuxième poulies 50', 64' étant fixes par rapport à l'axe d'articulation A1, l'on retrouve donc ici le même principe que dans le premier mode de réalisation et qui permet d'obtenir un couple de redressement suivant une loi sinusoïdale ou pseudo-sinusoïdale, comme on peut le voir sur la [Fig.16].

De plus, de manière analogue la troisième poulie 65' constitue un troisième point d'appui G2' pour l'élément de transmission 7' et le second point de fixation 63' constitue un quatrième point d'appui G3', dont les distances à l'axe d'articulation secondaire A3 sont également fixes. L'on retrouve donc là encore localement le même principe qu'entre le maillon 62' et le support supérieur 5'.

L'exosquelette E' a un encombrement réduit au niveau des fesses de l'utilisateur U, ce qui lui permet de s'asseoir plus facilement sur un siège, par exemple pour conduire un chariot élévateur, et l'exosquelette E' conserve les avantages procurés par un couple de redressement qui suit une loi pseudo-sinusoïdale dépendant, entre autres, de la force produite par l'organe élastiquement déformable et du sinus de l'angle formé entre la droite reliant l'axe d'articulation A1 et le premier point d'appui G0' et la droite reliant les premier et second points d'appui G0' et G1', comme dans le cas des supports supérieur et inférieur 5, 6 rigides.

On a illustré sur les Figures 13 à 16 un exosquelette E' comprenant un seul maillon 62'. Toutefois, l'on comprendra aisément qu'il est possible de prévoir un ensemble d'articulation à maillon(s) 61' comprend plusieurs tels maillons 62' entre le corps de base 60' et le support supérieur 5', à la manière d'une colonne vertébrale contenant plusieurs vertèbres, comme illustré sur la [Fig.17] pour une variante à deux maillons 62'a, 62'b.

Dans un tel cas, les maillons 62'a, 62'b successifs sont articulés entre eux autour d'axes de rotation A5 parallèles à l'axe d'articulation A1 et chaque maillon 62'a, 62'b portera encore deux poulies, une au voisinage de chaque extrémité du maillon 62'a, 62'b.

L'on comprendra aisément que, localement, entre deux maillons 62'a, 62'b successifs, l'on retrouve le même principe suivant lequel l'élément de transmission 7' est en appui contre deux points d'appui G4' et G5', l'un G4' formé par une poulie d'un maillon 62'a et l'autre G5' formé par une poulie du maillon 62'b suivant, dont les distances à l'axe d'articulation A5 entre les maillons 62'a, 62'b sont fixes.

Un tel exosquelette E' à plusieurs maillons 62'a, 62'b conservent donc également l'avantage d'un couple de redressement qui suit une loi sinusoïdale ou pseudo-sinusoïdale comme dans le premier mode de réalisation.

Il est bien entendu que les modes de réalisation particuliers qui viennent d'être décrits ont été donnés à titre indicatif et non limitatif, et que des modifications peuvent être apportées sans que l'on s'écarte pour autant du cadre de la présente invention.

## Revendications

1. Exosquelette (E ; E') d'assistance au maintien en posture penchée en avant et au redressement d'un utilisateur (U), l'exosquelette (E ; E') comprenant une structure dorsale (1 ; 1') configurée pour être fixée de façon amovible au niveau de la région dorsale de l'utilisateur (U) et deux structures de liaison de cuisse (2 ; 2') configurées pour être fixées de façon amovible aux cuisses de l'utilisateur (U) et reliées à la structure dorsale (1 ; 1') par l'intermédiaire d'un dispositif de génération de couple (3 ; 3') passif comprenant, pour chaque structure de liaison de cuisse (2 ; 2'), un support supérieur (5 ; 5') couplé à la structure dorsale (1 ; 1'), un support inférieur (6 ; 6') couplé à la structure de liaison de cuisse (2 ; 2'), le support supérieur (5 ; 5') et le support inférieur (6 ; 6') étant articulés l'un à l'autre en rotation, dans un même plan, autour d'un axe d'articulation (A1), le dispositif de génération de couple (3 ; 3') comprenant en outre, pour chaque structure de liaison de cuisse (2 ; 2'), un élément de transmission (7 ; 7') longitudinal, souple et inextensible, ayant une première extrémité reliée, en un premier point de fixation (63 ; 63'), au support inférieur (6 ; 6'), et une seconde extrémité reliée, en un second point de fixation (87), à une première partie, dite mobile, d'un organe élastiquement déformable (8 ; 8') dont une seconde partie, dite fixe, est solidaire en rotation du support supérieur (5 ; 5') de façon à tourner conjointement avec le support supérieur (5 ; 5') relativement au support inférieur (6 ; 6'), l'organe élastiquement déformable (8 ; 8') étant apte à produire une force rectiligne (F) le long d'un axe de déformation (A2) perpendiculaire à l'axe d'articulation (A1) et dont 1a grandeur est linéairement proportionnelle à sa déformation élastique (ΔL), l'élément de transmission (7 ; 7') étant par ailleurs en appui contre un premier point d'appui (G0 ; G0') solidaire du support supérieur (5 ; 5') et un second point d'appui (G1 ; G1') solidaire du support inférieur (6 ; 6'), chacun des premier et second points d'appui (G0, G1 ; G0', G1') se trouvant à une distance (Rh, Rb) fixe de l'axe d'articulation (A1) lors d'une rotation entre le support supérieur (5 ; 5') et le support inférieur (6 ; 6'), le dispositif de génération de couple (3 ; 3') étant ainsi apte, dans un mode de fonctionnement passif, lorsque l'utilisateur (U) se penche en avant, dans un plan perpendiculaire à l'axe d'articulation (A1), à produire un couple de redressement (C) appliqué entre la structure dorsale (1 ; 1') et chaque structure de liaison de cuisse (2 ; 2') du fait de la déformation de l'organe élastiquement déformable (8 ; 8') par l'élément de transmission (7 ; 7'), **caractérisé par le fait que** le couple de redressement (C) suit une loi pseudo-sinusoïdale qui est fonction de 1a force (F) produite par l'organe élastiquement déformable (8 ; 8') et du sinus d'un angle (β) formé entre la droite reliant l'axe d'articulation (A1) et le premier point d'appui (G0 ; G0') et la droite reliant les premier et second points d'appui (G0, G1 ; G0', G1').

2. Exosquelette (E ; E') selon 1a revendication 1, **caractérisé par le fait que** chaque élément de transmission (7 ; 7') est un câble, notamment un câble réalisé en fibre de polyéthylène haut module.

3. Exosquelette (E ; E') selon l'une quelconque des revendications 1 et 2, **caractérisé par le fait que** la longueur de chaque élément de transmission (7 ; 7') est telle qu'en utilisation, jusqu'à un angle d'inclinaison prédéterminé du dos de l'utilisateur (U) par rapport à l'arrière de ses cuisses, l'élément de transmission (7 ; 7') ne provoque pas de déformation de l'organe élastiquement déformable (8 ; 8').

4. Exosquelette (E) selon l'une quelconque des revendications 1 à 3, **caractérisé par le fait que** l'exosquelette (E) comprend, pour chaque élément de transmission (7), un module de mise en traction/détente auquel est relié l'élément de transmission (7), le module de mise en traction/détente étant actionnable par l'utilisateur (U) de façon à faire varier la longueur libre de l'élément de transmission (7), le module de mise en traction/détente étant mobile entre une position embrayée, dans laquelle l'élément de transmission est tendu, et une position débrayée, dans laquelle l'élément de transmission est détendu, de telle sorte qu'il ne provoque pas de déformation de l'organe élastiquement déformable (8).

5. Exosquelette (E) selon la revendication 4, **caractérisé par le fait que** le module de mise en traction/détente est formé par un tambour d'enroulement (64) sur lequel le premier point de fixation (63) est positionné, le tambour d'enroulement (64) étant relié au support inférieur (6) de façon à être apte à tourner autour d'un axe parallèle audit axe d'articulation (A1) et ainsi à enrouler ou dérouler l'élément de transmission (7), selon le sens de rotation, le module de mise en traction/détente comprenant en outre des moyens de blocage du module de mise en traction/détente dans chacune des positions embrayée et débrayée.

6. Exosquelette (E) selon l'une quelconque des revendications 1 à 5, **caractérisé par** le fait chaque support inférieur (6) comporte une fourchette de réglage de couple (9) sur laquelle est positionné le second point d'appui (G1), et dont la position est réglable par rapport à l'axe d'articulation (A1), de telle sorte que le réglage de la position de la fourchette (9) permet de modifier la distance (Rb) entre l'axe d'articulation (A1) et le second point d'appui (G1).

7. Exosquelette (E ; E') selon l'une quelconque des revendications 1 à 6, **caractérisé par le fait que** le dispositif de génération de couple (3 ; 3') comprend deux organes élastiquement déformables (8 ; 8'), à savoir un organe élastiquement déformable (8 ; 8') pour chaque structure de liaison de cuisse (2 ; 2').

8. Exosquelette (E ; E') selon la revendication 7, **caractérisé par le fait que** chaque organe élastiquement déformable (8 ; 8') comprend un ressort de compression (8 ; 8'), chaque ressort de compression (8 ; 8') ayant une extrémité inférieure (8b) qui constitue ladite partie fixe et est fixée au support supérieur (5 ; 5'), et une extrémité supérieure (8a) qui constitue ladite partie mobile est reliée à l'élément de transmission (7 ; 7'), au niveau du second point de fixation (87), chaque ressort (8 ; 8') s'étendant selon un axe de déformation (A2) parallèle à l'axe de la structure dorsale (1 ; 1') et perpendiculaire à l'axe d'articulation (A1), de telle sorte qu'en utilisation, lorsque l'utilisateur (U) se penche en avant, le second point de fixation (87) de l'élément de transmission (7 ; 7') se déplace en direction de l'extrémité inférieure (8b) du ressort (8 ; 8') d'une distance (ΔL), provoquant la compression du ressort (8 ; 8'), qui amène le ressort (8 ; 8') à exercer une force de rappel (F) axiale proportionnelle à la distance de déplacement (ΔL).

9. Exosquelette (E) selon l'une quelconque des revendications 1 à 8, **caractérisé par le fait que** l'exosquelette (E) comprend un outre un module électronique de pilotage du couple de redressement, comprenant des moyens de détermination (CI) de l'angle d'inclinaison du buste de l'utilisateur, un actionneur motorisé (M, V) configuré pour commander, lors de son actionnement, dans un mode de fonctionnement dit quasi-passif de l'exosquelette (E), la déformation de l'organe élastiquement déformable (8), indépendamment d'un quelconque pivotement relatif entre les supports supérieur (5) et inférieur (6), des moyens de commande de l'actionneur sur la base de l'angle d'inclinaison déterminé par lesdits moyens de détermination (CI), et des moyens de maintien de l'organe élastiquement déformable (8) dans sa configuration déformée par l'actionneur.

10. Exosquelette (E) selon l'une quelconque des revendications 1 à 9, **caractérisé par le fait que** la structure dorsale (1) comprend deux paires de tubes télescopiques dorsaux (11), chaque paire (11) comprenant un tube inférieur (11b) et un tube supérieur (11a) apte à coulisser dans le tube inférieur (11b), le tube inférieur (11b) de chaque paire (11) étant solidaire du support supérieur (5) correspondant, le tube supérieur (11a) de chaque paire (11) étant couplé à des moyens de fixation amovible (12) à l'épaule de l'utilisateur (U), les deux paires (11) s'étendant selon des axes parallèles entre eux et parallèles à l'axe de déformation (A2).

11. Exosquelette (E) selon l'une quelconque des revendications 1 à 10, **caractérisé par le fait que** chaque structure liaison de cuisse (2) comprend une paire de tubes télescopiques de cuisse (20), à savoir un tube supérieur (20a) et un tube inférieur (20b) apte à coulisser dans le tube supérieur (20a), le tube supérieur (20a) étant solidaire du support inférieur (6) correspondant et le tube inférieur (20b) étant couplé à des moyens de fixation amovible (21) à la cuisse de l'utilisateur (U).

12. Exosquelette (E') selon l'une quelconque des revendications 1 à 11, **caractérisé par le fait que** chaque support inférieur (6') est un support articulé, comprenant un corps de base (60'), couplé à la structure de liaison de cuisse (2') et auquel se situe le premier point de fixation (63') de l'élément de transmission (7'), et un ensemble d'articulation à maillon(s) (61') dont une première extrémité est articulée en rotation au support supérieur (5') autour de l'axe d'articulation (A1) et dont une seconde extrémité est articulée en rotation au corps de base (60') autour d'un axe d'articulation secondaire (A3) parallèle audit axe d'articulation (A1), le second point d'appui (G1') étant solidaire de l'ensemble d'articulation à maillon(s) (61'), dans la région de sa première extrémité, l'élément de transmission (7') étant par ailleurs en appui contre un troisième point d'appui (G2') solidaire de l'ensemble d'articulation à maillon(s) (61'), dans la région de sa seconde extrémité, et contre un quatrième point d'appui (G3') solidaire du corps de base (60'), chacun des troisième et quatrième points d'appui (G2', G3') se trouvant à une distance fixe de l'axe d'articulation secondaire (A3) lors d'une rotation entre le support supérieur (5') et le support inférieur (6').

13. Exosquelette (E') selon la revendication 12, **caractérisé par le fait que** chaque ensemble d'articulation à maillon(s) (61') comprend un seul maillon (62') rigide dont une première extrémité est articulée en rotation au support supérieur (5') autour de l'axe d'articulation (A1) et dont une seconde extrémité est articulée en rotation au corps de base (60') autour d'un axe d'articulation secondaire (A3) parallèle audit axe d'articulation (A1), le maillon (62') portant les second et troisième points d'appui (G1', G2').

14. Exosquelette (E') selon la revendication 12, **caractérisé par le fait que** chaque ensemble d'articulation à maillon(s) (61') comprend au moins deux maillons (62'a, 62'b) rigides successifs articulés entre eux, un premier des maillons (62'a, 62'b), dit maillon supérieur (62'a), ayant une première extrémité articulée en rotation au support supérieur (5') autour de l'axe d'articulation (A1), et un second des maillons (62'a, 62'b), dit maillon inférieur (62'b), ayant une seconde extrémité articulée en rotation au corps de base (60') autour d'un axe d'articulation secondaire (A3) parallèle audit axe d'articulation (A1), chaque maillon (62'a, 62'b) étant par ailleurs articulé en rotation au maillon suivant autour d'un axe d'articulation intermédiaire (A5) parallèle audit axe d'articulation (A1), le maillon supérieur (62'a) portant le second point d'appui (G1') et le maillon inférieur (62'b) portant le troisième point d'appui (G2'), l'élément de transmission (7') étant par ailleurs en appui, pour chaque paire de maillons (62'a, 62'b) successifs, composée d'un premier maillon (62'a) et d'un second maillon (62'b), contre un premier point d'appui intermédiaire (G4') solidaire du premier maillon (62'a) de ladite paire, dans la région de sa seconde extrémité, et contre un second point d'appui intermédiaire (G5') solidaire du second maillon (62'b) de ladite paire, dans la région de sa première extrémité, chacun des premier et second points d'appui intermédiaires (G4', G5') se trouvant à une distance fixe de l'axe d'articulation intermédiaire (A5) entre les premier et second maillons (62'a, 62'b) de ladite paire lors d'une rotation entre le support supérieur (5') et le support inférieur (6').

## Patentansprüche

1. - Exoskelett (E; E') zur Unterstützung des Haltens einer nach vorne gebeugten Haltung und des Aufrichtens eines Benutzers (U), wobei das Exoskelett (E; E') eine Rückenstruktur (1; 1'), die so konfiguriert ist, dass sie lösbar am Rückenbereich des Benutzers (U) befestigt ist, und zwei Oberschenkelverbindungsstrukturen (2; 2') umfasst, die so konfiguriert sind, dass sie lösbar an den Oberschenkeln des Benutzers (U) befestigt sind und mit der Rückenstruktur (1; 1') über eine passive Drehmomenterzeugungsvorrichtung (3; 3') verbunden sind, die für jede Oberschenkelverbindungsstruktur (2; 2') eine obere Halterung (5; 5'), die mit der Rückenstruktur (1; 1') gekoppelt ist, eine untere Halterung (6; 6'), die mit der Oberschenkelverbindungsstruktur (2; 2') gekoppelt ist, umfasst, wobei die obere Halterung (5 ; 5') und die untere Halterung (6; 6') in einer gleichen Ebene um eine Gelenkachse (A1) drehbar aneinander angelenkt sind, wobei die Drehmomenterzeugungsvorrichtung (3; 3') für jede Oberschenkelverbindungsstruktur (2; 2') außerdem ein längliches, flexibles und undehnbares Übertragungselement (7; 7') umfasst, das ein erstes Ende hat, das an einem ersten Befestigungspunkt (63 ; 63') mit der unteren Halterung (6; 6') verbunden ist, und ein zweites Ende, das an einem zweiten Befestigungspunkt (87) mit einem ersten, sogenannten beweglichen Teil eines elastisch verformbaren Organs (8; 8') verbunden ist, von dem ein zweiter, sogenannter fester Teil drehfest mit der oberen Halterung (5; 5') verbunden ist, um sich gemeinsam mit der oberen Halterung (5; 5') relativ zu der unteren Halterung (6; 6') zu drehen; wobei das elastisch verformbare Organ (8; 8') geeignet ist, eine geradlinige Kraft (F) entlang einer Verformungsachse (A2) zu erzeugen, die senkrecht zur Gelenkachse (A1) verläuft und deren Größe linear proportional zu seiner elastischen Verformung (ΔL) ist, wobei das Übertragungselement (7; 7') stützt sich außerdem an einem ersten Stützpunkt (G0 ; G0'), der fest mit der oberen Halterung (5; 5') verbunden ist, und einem zweiten Stützpunkt (G1; G1'), der fest mit der unteren Halterung (6; 6') verbunden ist, wobei sich jeder der ersten und zweiten Stützpunkte (G0, G1; G0', G1') bei einer Drehung zwischen der oberen Halterung (5; 5') und der unteren Halterung (6; 6') in einem festen Abstand (Rh, Rb) von der Gelenkachse (A1) befindet, wobei die Drehmomenterzeugungsvorrichtung (3 ; 3') somit in einem passiven Betriebsmodus, wenn sich der Benutzer (U) in einer Ebene senkrecht zur Gelenkachse (A1) nach vorne beugt, geeignet ist, ein aufrichtendes Drehmoment (C) zu erzeugen, das zwischen der Rückenstruktur (1; 1') und jeder Oberschenkelverbindungsstruktur (2; 2') aufgrund der Verformung des elastisch verformbaren Organs (8; 8') durch das Übertragungselement (7 ; 7') wirkt, **dadurch gekennzeichnet, dass** das aufrichtende Drehmoment (C) einem pseudosinusförmigen Gesetz folgt, das eine Funktion der Kraft (F), die durch das elastisch verformbare Organ (8; 8') erzeugt wird, und des Sinus eines Winkels (β) ist, der zwischen der Geraden, die die Gelenkachse (A1) und den ersten Stützpunkt (G0; G0') verbindet, und der Geraden, die den ersten und den zweiten Stützpunkt (G0, G1; G0', G1') verbindet, gebildet wird.

2. - Exoskelett (E; E') nach Anspruch 1, **dadurch gekennzeichnet, dass** jedes Übertragungselement (7; 7') ein Kabel ist, insbesondere ein Kabel, das aus hochmoduliger Polyethylenfaser hergestellt ist.

3. - Exoskelett (E; E') nach einem der Ansprüche 1 oder 2, **dadurch gekennzeichnet, dass** die Länge jedes Übertragungselements (7; 7') derart ist, dass im Gebrauch bis zu einem vorbestimmten Neigungswinkel des Rückens des Benutzers (U) in Bezug auf die Rückseite seiner Oberschenkel das Übertragungselement (7; 7') keine Verformung des elastisch verformbaren Organs (8; 8') bewirkt.

4. - Exoskelett (E) nach einem der Ansprüche 1 bis 3, **dadurch gekennzeichnet, dass** das Exoskelett (E) für jedes Übertragungselement (7) ein Zug-/Entspannungsmodul umfasst, mit dem das Übertragungselement (7) verbunden ist, wobei das Zug-/Entspannungsmodul durch den Benutzer (U) betätigbar ist, um die freie Länge des Übertragungselements (7) zu variieren, wobei das Zug-/Entspannungsmodul zwischen einer eingekuppelten Position, in der das Übertragungselement gespannt ist, und einer ausgekuppelten Position, in der das Übertragungselement entspannt ist, beweglich ist, so dass es keine Verformung des elastisch verformbaren Organs (8) bewirkt.

5. - Exoskelett (E) nach Anspruch 4, **dadurch gekennzeichnet, dass** das Zug-/Entspannungsmodul durch eine Wickeltrommel (64) gebildet wird, auf der der erste Befestigungspunkt (63) positioniert ist, die Wickeltrommel (64) mit der unteren Halterung (6) so verbunden ist, dass sie sich um eine Achse parallel zur Gelenkachse (A1) drehen kann und somit das Übertragungselement (7) je nach Drehrichtung auf- oder abwickelt, wobei das Zug-/Entspannungsmodul außerdem Mittel zum Blockieren des Zug- /Entspannungsmoduls in jeder der eingekuppelten und ausgekuppelten Positionen umfasst.

6. - Exoskelett (E) nach einem der Ansprüche 1 bis 5, **dadurch gekennzeichnet, dass** jede untere Halterung (6) eine Drehmomenteinstellgabel (9) aufweist, auf der der zweite Stützpunkt (G1) positioniert ist, und deren Position in Bezug auf die Gelenkachse (A1) einstellbar ist, so dass die Einstellung der Position der Gabel (9) es ermöglicht, den Abstand (Rb) zwischen der Gelenkachse (A1) und dem zweiten Stützpunkt (G1) zu verändern.

7. - Exoskelett (E; E') nach einem der Ansprüche 1 bis 6, **dadurch gekennzeichnet, dass** die Drehmomenterzeugungsvorrichtung (3; 3') zwei elastisch verformbare Organe (8; 8') umfasst, nämlich ein elastisch verformbares Organ (8; 8') für jede Oberschenkelverbindungsstruktur (2; 2').

8. - Exoskelett (E; E') nach Anspruch 7, **dadurch gekennzeichnet, dass** jedes elastisch verformbare Organ (8; 8') eine Druckfeder (8; 8') umfasst, wobei jede Druckfeder (8; 8') ein unteres Ende (8b) hat, das den festen Teil bildet und an der oberen Halterung (5; 5') befestigt ist, und ein oberes Ende (8a), das den beweglichen Teil bildet und an dem zweiten Befestigungspunkt (87) mit dem Übertragungselement (7; 7') verbunden ist, wobei jede Feder (8 ; 8') sich entlang einer Verformungsachse (A2) parallel zur Achse der Rückenstruktur (1; 1') und senkrecht zur Gelenkachse (A1) erstreckt, so dass im Gebrauch, wenn sich der Benutzer (U) nach vorne beugt, der zweite Befestigungspunkt (87) des Übertragungselements (7 ; 7') in Richtung des unteren Endes (8b) der Feder (8; 8') um eine Strecke (ΔL) verschoben wird, wodurch die Feder (8; 8') komprimiert wird, was die Feder (8; 8') dazu veranlasst, eine axiale Rückstellkraft (F) auszuüben, die proportional zur Verschiebungsstrecke (ΔL) ist.

9. - Exoskelett (E) nach einem der Ansprüche 1 bis 8, **dadurch gekennzeichnet, dass** das Exoskelett (E) zusätzlich ein elektronisches Modul zur Steuerung des aufrichtenden Drehmoments, das Mittel zur Bestimmung (CI) des Neigungswinkels des Oberkörpers des Benutzers umfasst, einen motorisierten Aktuator (M, V) umfasst, der so konfiguriert ist, dass er bei seiner Betätigung in einem sogenannten quasi-passiven Betriebsmodus des Exoskeletts (E) steuert, die Verformung des elastisch verformbaren Organs (8) unabhängig von einer beliebigen relativen Schwenkung zwischen der oberen (5) und der unteren (6) Halterung, Mittel zur Steuerung des Aktuators auf der Grundlage des durch die Bestimmungsmittel (CI) bestimmten Neigungswinkels, und Mittel zum Halten des elastisch verformbaren Organs (8) in seiner durch den Aktuator verformten Konfiguration.

10. - Exoskelett (E) nach einem der Ansprüche 1 bis 9, **dadurch gekennzeichnet, dass** die Rückenstruktur (1) zwei Paare von teleskopischen Rückenrohren (11) umfasst, wobei jedes Paar (11) ein unteres Rohr (11b) und ein oberes Rohr (11a) umfasst, das in dem unteren Rohr (11b) gleiten kann, das untere Rohr (11b) jedes Paares (11) fest mit der entsprechenden oberen Halterung (5) verbunden ist, das obere Rohr (11a) jedes Paares (11) mit Mitteln zur lösbaren Befestigung (12) an der Schulter des Benutzers (U) gekoppelt ist, wobei sich die beiden Paare (11) entlang Achsen erstrecken, die parallel zueinander und parallel zur Deformationsachse (A2) sind.

11. - Exoskelett (E) nach einem der Ansprüche 1 bis 10, **dadurch gekennzeichnet, dass** jede Oberschenkelverbindungsstruktur (2) ein Paar teleskopische Oberschenkelrohre (20) umfasst, nämlich ein oberes Rohr (20a) und ein unteres Rohr (20b), das in dem oberen Rohr (20a) gleiten kann, wobei das obere Rohr (20a) fest mit der entsprechenden unteren Halterung (6) verbunden ist und das untere Rohr (20b) mit Mitteln zur lösbaren Befestigung (21) am Oberschenkel des Benutzers (U) gekoppelt ist.

12. - Exoskelett (E') nach einem der Ansprüche 1 bis 11, **dadurch gekennzeichnet, dass** jede untere Halterung (6') eine Gelenkhalterung ist, die einen Grundkörper (60') umfasst, der mit der Oberschenkelverbindungsstruktur (2') gekoppelt ist und an dem sich der erste Befestigungspunkt (63') des Elements zur Übertragung (7') befindet, und eine Gelenkeinheit mit Glied(ern) (61'), deren erstes Ende an der oberen Halterung (5') um die Gelenkachse (A1) drehbar angelenkt ist und deren zweites Ende an dem Grundkörper (60') um eine sekundäre Gelenkachse (A3) parallel zu der Gelenkachse (A1) drehbar angelenkt ist, wobei der zweite Stützpunkt (G1') im Bereich seines ersten Endes fest mit der Gelenkeinheit mit Glied(ern) (61') verbunden ist, wobei das Übertragungselement (7') außerdem an einem dritten Stützpunkt (G2') anliegt, der fest mit der Gelenkeinheit mit Glied(ern) (61') verbunden ist, im Bereich seines zweiten Endes und gegen einen vierten Stützpunkt (G3'), der fest mit dem Grundkörper (60') verbunden ist, wobei sich jeder der dritten und vierten Stützpunkte (G2', G3') bei einer Drehung zwischen der oberen Halterung (5') und der unteren Halterung (6') in einem festen Abstand von der sekundären Gelenkachse (A3) befindet.

13. - Exoskelett (E') nach Anspruch 12, **dadurch gekennzeichnet, dass** jede Gelenkeinheit mit Glied(ern) (61') ein einziges starres Glied (62') umfasst, dessen erstes Ende um die Gelenkachse (A1) drehbar an der oberen Halterung (5') angelenkt ist und dessen zweites Ende um eine sekundäre Gelenkachse (A3), die parallel zur Gelenkachse (A1) verläuft, drehbar am Grundkörper (60') angelenkt ist, wobei das Glied (62') den zweiten und dritten Stützpunkt (G1', G2') trägt.

14. - Exoskelett (E') nach Anspruch 12, **dadurch gekennzeichnet, dass** jede Gelenkeinheit mit Glied(ern) (61') mindestens zwei aufeinanderfolgende starre Glieder (62'a, 62'b) umfasst, die gelenkig miteinander verbunden sind, wobei ein erstes der Glieder (62'a, 62'b), das so genannte obere Glied (62'a), ein erstes Ende hat, das um die Gelenkachse (A1) drehbar an der oberen Halterung (5') angelenkt ist, und ein zweites der Glieder (62'a, 62'b), das sogenannte untere Glied (62'b), mit einem zweiten Ende, das um eine sekundäre Gelenkachse (A3) parallel zur Gelenkachse (A1) drehbar am Grundkörper (60') angelenkt ist, wobei jedes Glied (62'a, 62'b) außerdem um eine mittlere Gelenkachse (A5) parallel zur Gelenkachse (A1) drehbar am nächsten Glied angelenkt ist, wobei das obere Glied (62'a) den zweiten Stützpunkt (G1') trägt und das untere Glied (62'b) den dritten Stützpunkt (G2') trägt, wobei das Übertragungselement (7') außerdem für jedes Paar von aufeinanderfolgenden Glieder (62'a, 62'b), das aus einem ersten Glied (62'a) und einem zweiten Glied (62'b) besteht, gegen einen ersten Zwischenstützpunkt (G4'), der fest mit dem ersten Glied (62'a) des Paares verbunden ist, im Bereich seines zweiten Endes anliegt, und gegen einen zweiten Zwischenstützpunkt (G5'), der fest mit dem zweiten Glied (62'b) des Paares verbunden ist, im Bereich seines ersten Endes, wobei sich jeder der ersten und zweiten Zwischenstützpunkte (G4', G5') bei einer Drehung zwischen der oberen Halterung (5') und der unteren Halterung (6') in einem festen Abstand von der Zwischengelenkachse (A5) zwischen dem ersten und dem zweiten Glied (62'a, 62'b) des Paares befindet.

## Claims

1. - Exoskeleton (E; E') for assisting a user (U) in maintaining a forward-leaning posture and in straightening up, the exoskeleton (E; E') comprising a dorsal structure (1; 1') configured to be removably attached at the dorsal region of the user (U) and two thigh connection structures (2; 2') configured to be removably attached to the thighs of the user (U) and connected to the dorsal structure (1; 1') by means of a passive torque generation device (3; 3') comprising, for each thigh connection structure (2; 2'), an upper support (5; 5') coupled to the dorsal structure (1; 1'), a lower support (6; 6') coupled to the thigh connection structure (2; 2'), the upper support (5; 5') and the lower support (6; 6') being rotatably articulated to each other, in the same plane, about an articulation axis (A1), the torque generating device (3; 3') further comprising, for each thigh connection structure (2; 2'), a longitudinal, flexible and inextensible transmission element (7; 7') having a first end connected, at a first fixing point (63; 63'), to the lower support (6; 6') and a second end connected, at a second fixing point (87), to a first, so-called mobile, part of an elastically deformable member (8; 8') of which a second, so-called fixed, part is rotationally fixed to the upper support (5; 5') so as to rotate together with the upper support (5; 5') relative to the lower support (6; 6'), the elastically deformable member (8; 8') being capable of producing a rectilinear force (F) along a deformation axis (A2) perpendicular to the articulation axis (A1) and whose magnitude is linearly proportional to its elastic deformation (ΔL), the transmission element (7; 7') also bearing against a first bearing point (G0; G0') integral with the upper support (5; 5') and a second bearing point (G1; G1') integral with the lower support (6; 6'), each of the first and second bearing points (G0, G1; G0', G1') being at a fixed distance (Rh, Rb) from the articulation axis (A1) during rotation between the upper support (5; 5') and the lower support (6; 6'), the torque generation device (3; 3') being thus capable, in a passive mode of operation, when the user (U) leans forward, in a plane perpendicular to the articulation axis (A1), of producing a straightening torque (C) applied between the dorsal structure (1; 1') and each thigh connection structure (2; 2') due to the deformation of the elastically deformable member (8; 8') by the transmission element (7; 7'), **characterised in that** the straightening torque (C) follows a pseudo-sinusoidal law that is a function of the force (F) produced by the elastically deformable member (8; 8') and the sine of an angle (β) formed between the straight line connecting the articulation axis (A1) and the first bearing point (G0; G0') and the straight line connecting the first and second bearing points (G0, G1; G0', G1').

2. - Exoskeleton (E; E') according to claim 1, **characterised in that** each transmission element (7; 7') is a cable, in particular a cable made of high modulus polyethylene fibre.

3. - Exoskeleton (E; E') according to any of claims 1 and 2, **characterised in that** the length of each transmission element (7; 7') is such that, in use, up to a predetermined angle of inclination of the back of the user (U) relative to the back of their thighs, the transmission element (7; 7') does not cause deformation of the elastically deformable member (8; 8').

4. - Exoskeleton (E) according to any of claims 1 to 3, **characterised in that** the exoskeleton (E) comprises, for each transmission element (7), a tension/release module to which the transmission element (7) is connected, the tension/release module being operable by the user (U) so as to vary the free length of the transmission element (7), the tension/release module being movable between an engaged position, in which the transmission element is tensioned, and a disengaged position, in which the transmission element is released, so that it does not cause deformation of the elastically deformable member (8).

5. - Exoskeleton (E) according to claim 4, **characterised in that** the tension/release module is formed by a winding drum (64) on which the first fixing point (63) is positioned, the winding drum (64) being connected to the lower support (6) so as to be able to rotate about an axis parallel to said articulation axis (A1) and thus wind or unwind the transmission element (7), depending on the direction of rotation, the tension/release module further comprising means for locking the tension/release module in each of the engaged and disengaged positions.

6. - Exoskeleton (E) according to any of claims 1 to 5, **characterised in that** each lower support (6) comprises a torque adjustment fork (9) on which the second bearing point (G1) is positioned, and whose position is adjustable relative to the articulation axis (A1), such that adjusting the position of the fork (9) allows the distance (Rb) between the articulation axis (A1) and the second bearing point (G1) to be modified.

7. - Exoskeleton (E; E') according to any of claims 1 to 6, **characterised in that** the torque generation device (3; 3') comprises two elastically deformable members (8; 8'), namely one elastically deformable member (8; 8') for each thigh connection structure (2; 2').

8. - Exoskeleton (E; E') according to claim 7, **characterised in that** each elastically deformable member (8; 8') comprises a compression spring (8; 8'), each compression spring (8; 8') having a lower end (8b) that constitutes said fixed part and is fixed to the upper support (5; 5'), and an upper end (8a) that constitutes said movable part and is connected to the transmission element (7; 7'), at the second fixing point (87), each spring (8; 8') extending along a deformation axis (A2) parallel to the axis of the dorsal structure (1; 1') and perpendicular to the articulation axis (A1), such that, in use, when the user (U) leans forward, the second fixing point (87) of the transmission element (7; 7') moves towards the lower end (8b) of the spring (8; 8') by a distance (ΔL), causing the spring (8; 8') to compress, which causes the spring (8; 8') to exert an axial restoring force (F) proportional to the distance of displacement (ΔL).

9. - Exoskeleton (E) according to any of claims 1 to 8, **characterised in that** the exoskeleton (E) further comprises an electronic module for controlling the straightening torque, comprising means (CI) for determining the angle of inclination of the user's torso, a motorised actuator (M, V) configured to control, when actuated, in a so-called quasi-passive mode of operation of the exoskeleton (E), the deformation of the elastically deformable member (8), independently of any relative pivoting between the upper (5) and lower (6) supports, means for controlling the actuator on the basis of the angle of inclination determined by said determination means (CI), and means for maintaining the elastically deformable member (8) in its configuration deformed by the actuator.

10. - Exoskeleton (E) according to any of claims 1 to 9, **characterised in that** the dorsal structure (1) comprises two pairs of dorsal telescopic tubes (11), each pair (11) comprising a lower tube (11b) and an upper tube (11a) capable of sliding in the lower tube (11b), the lower tube (11b) of each pair (11) being secured to the corresponding upper support (5), the upper tube (11a) of each pair (11) being coupled to means (12) for removable attachment to the shoulder of the user (U), the two pairs (11) extending along axes that are parallel to each other and parallel to the deformation axis (A2).

11. - Exoskeleton (E) according to any of claims 1 to 10, **characterised in that** each thigh connection structure (2) comprises a pair of telescopic thigh tubes (20), namely an upper tube (20a) and a lower tube (20b) capable of sliding in the upper tube (20a), the upper tube (20a) being secured to the corresponding lower support (6) and the lower tube (20b) being coupled to means (21) for removable attachment to the thigh of the user (U).

12. - Exoskeleton (E') according to any one of claims 1 to 11, **characterised in that** each lower support (6') is an articulated support, comprising a base body (60') coupled to the thigh connection structure (2') and at which the first fixing point (63') of the transmission element (7'), and a link joint assembly (61') whose first end is rotatably articulated to the upper support (5') about the articulation axis (A1) and whose second end is rotatably articulated to the base body (60') around a secondary articulation axis (A3) parallel to said articulation axis (A1), the second bearing point (G1') being integral with the link assembly (61'), in the region of its first end, the transmission element (7') also bearing against a third bearing point (G2') integral with the link joint assembly (61'), in the region of its second end, and against a fourth bearing point (G3') integral with the base body (60'), each of the third and fourth bearing points (G2', G3') being at a fixed distance from the secondary articulation axis (A3) during rotation between the upper support (5') and the lower support (6').

13. - Exoskeleton (E') according to claim 12, **characterised in that** each link joint assembly (61') comprises a single rigid link (62') a first end of which is rotatably articulated to the upper support (5') about the articulation axis (A1) and a second end of which is rotatably articulated to the base body (60') about a secondary articulation axis (A3) parallel to said articulation axis (A1), the link (62') carrying the second and third bearing points (G1', G2').

14. - Exoskeleton (E') according to claim 12, **characterised in that** each link joint assembly (61') comprises at least two successive rigid links (62'a, 62'b) articulated to each other, a first of the links (62'a, 62'b), so-called upper link (62'a), having a first end rotatably articulated to the upper support (5') about the articulation axis (A1), and a second of the links (62'a, 62'b), so-called the lower link (62'b), having a second end rotatably articulated to the base body (60') about a secondary articulation axis (A3) parallel to said articulation axis (A1), each link (62'a, 62'b) also being rotatably articulated to the next link about an intermediate articulation axis (A5) parallel to said articulation axis (A1), the upper link (62'a) carrying the second bearing point (G1') and the lower link (62'b) carrying the third bearing point (G2'), the transmission element (7') also bearing, for each pair of successive links (62'a, 62'b) composed of a first link (62'a) and a second link (62'b), against a first intermediate bearing point (G4') integral with the first link (62'a) of said pair, in the region of its second end, and against a second intermediate bearing point (G5') integral with the second link (62'b) of said pair, in the region of its first end, each of the first and second intermediate bearing points (G4', G5') being at a fixed distance from the intermediate articulation axis (A5) between the first and second links (62'a, 62'b) of said pair during rotation between the upper support (5') and the lower support (6').
